# EUROPÄISCHE PATENTSCHRIFT

(11) **EP 0 981 542 B1**
(45) Veröffentlichungstag und Bekanntmachung des Hinweises auf die Patenterteilung: **04.08.2004**
(21) Anmeldenummer: 98925527.8
(22) Anmeldetag: 04.05.1998
(51) Int. Cl.: C07K 9/00, C07H 15/203, C07D 491/22, A61K 38/14, A61K 31/70

(54) **GLYCOKONJUGATE VON 20(S)-CAMPTOTHECIN**
20(S) CAMPTOTHECIN GLYCOCONJUGATES
GLYCOCONJUGUES DE 20(S)-CAMPTOTHECINE

(30) Priorität: 14.05.1997 DE 19720043; 28.08.1997 DE 19737477; 14.01.1998 DE 19801037; 25.03.1998 DE 19813137
(43) Veröffentlichungstag der Anmeldung: 01.03.2000
(73) Patentinhaber: Bayer HealthCare AG, 51368 Leverkusen (DE)
(72) Erfinder: LERCHEN, Hans-Georg, D-51375 Leverkusen (DE); VON DEM BRUCH, Karsten, D-51375 Leverkusen (DE); BAUMGARTEN, Jörg, D-42115 Wuppertal (DE); SPERZEL, Michael, D-42275 Wuppertal (DE)
(86) Internationale Anmeldenummer: PCT/EP1998/002620
(87) Internationale Veröffentlichungsnummer: WO 1998/051703

(56) Entgegenhaltungen:
- EP-A- 0 501 250
- EP-A- 0 757 049
- WO-A-96/31532
- WO-A-98/14459
- WO-A-98/15573

## Beschreibung

Die vorliegende Erfindung betrifft Glycokonjugate von 20(S)-Camptothecin, in denen ein 3-O-methylierter β-L-Fucose-Baustein über Thioharnstoff-modifizierte Peptidspacer mit der 20-Hydroxylgruppe eines Camptothecin-Derivats verknüpft ist. Die Erfindung betrifft weiterhin Verfahren zur Herstellung der erfindungsgemäßen Verbindungen sowie deren Verwendung als Arzneimittel, insbesondere im Zusammenhang mit Krebserkrankungen.

20(S)-Camptothecin ist ein pentacyclisches Alkaloid, das 1966 von Wall et al. isoliert wurde (J.Am.Chem.Soc. 88, 3888 (1966)). Es besitzt ein hohes Antitumor-Wirkpotential in zahlreichen In-vitro- und In-vivo-Tests. Leider scheiterte jedoch die Realisierung des vielversprechenden Potentials in der Klinik an Toxizitäts- und Löslichkeitsproblemen.

Durch Öffnung des E-Ring-Lactons und Bildung des Natriumsalzes wurde eine wasserlösliche Verbindung erhalten, die in einem pH-abhängigen Gleichgewicht mit der ringgeschlossenen Form steht. Klinische Studien führten auch hier bisher nicht zum Erfolg.

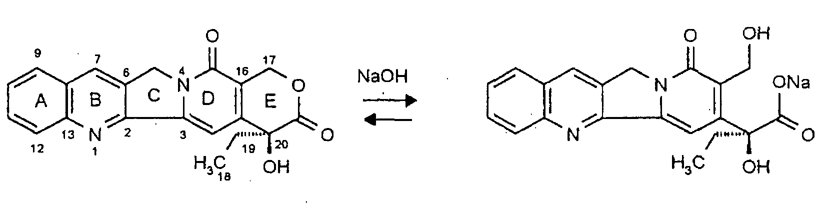

Etwa 20 Jahre später wurde gefunden, daß die biologische Aktivität auf eine Enzyminhibition der Topoisomerase I zurückzuführen ist. Seither wurden die Forschungsaktivitäten wieder verstärkt, um verträglichere und in-vivo wirksame Camptothecin-Derivate zu finden.

Zur Verbesserung der Wasserlöslichkeit wurden Salze von A-Ring- und B-Ringmodifizierten Camptothecin-Derivaten sowie von 20-O-Acyl-Derivaten mit ionisierbaren Gruppen beschrieben (Vishnuvajjala et al. US 4943579). Letzteres Prodrug-Konzept wurde später auch auf modifizierte Camptothecin-Derivate übertragen (Wani et al. WO 9602546). Die beschriebenen 20-O-Acyl-Prodrugs haben allerdings in-vivo eine sehr kurze Halbwertszeit und werden sehr schnell zum Grundkörper gespalten.

In WO 9631532 A1 wurden zuckermodifizierte Cytostatika beschrieben, bei denen die Verknüpfung von verschiedenen cytotoxischen oder cytostatisch aktiven Verbindungen mit z.B. regioselektiv modifizierten Kohlenhydratbausteinen über bestimmte Spacer zu einer Verbesserung der Tumorselektivität führte. Aus den dort breit beschriebenen Kombinationen von Kohlenhydrat, Spacer und Wirkstoff fanden wir nun überraschend, daß die Anknüpfung von in 3-Stellung modifizierten β-L-Fucosebausteinen über einen Thioharnstoff-modifizierten Peptid-Spacer, bestehend aus einer sterisch anpruchsvollen unpolaren und einer basischen Aminosäure an die 20-Hydroxyl-Gruppe von 20(S)-Camptothecin zu ganz besonders bevorzugten Konjugaten mit folgenden Eigenschaften führt:
- Durch die esterartige Anknüpfung des Carrier-Rests an die 20-Hydroxylgruppe wird der fur die Wirkung wichtige Lactonring im Camptothecin-Teil stabilisiert.
- Durch die spezielle Konstellation der Dipeptid-Spacer weisen die Konjugate in extrazellulärem Medium und in Blut eine im Vergleich zu ähnlichen, zuvor in WO 9631532 beschriebenen Konjugaten mit anderen Spacern nochmals deutlich verbesserte Stabilität auf. Insbesondere sind die erfindungsgemäßen Konjugate stabiler als die in US 4943579 beschriebenen 20-O-Acyl-Prodrugs von Camptothecin.
- Die erfindungsgemäßen Konjugate sind im Vergleich zu ähnlichen Konjugaten aus WO 9631532 besser wasserlöslich.
- Die erfindungsgemäßen Konjugate zeigen in-vitro eine hohe Aktivität gegenüber Tumorzellinien und Tumorxenografts.
- Die erfindungsgemäßen Konjugate zeigen in-vivo nach i v.-Applikation exzellente therapeutische Wirksamkeit über mehrere Dosisstufen gegenüber verschiedenen Tumoren.
- Sie weisen gegenüber dem zugrundeliegenden Toxophor eine deutlich höhere Verträglichkeit und Tumorselektivität insbesondere im Hinblick auf Knochenmarktoxizität auf.

Die Erfindung betrifft Verbindungen der Formel (I) 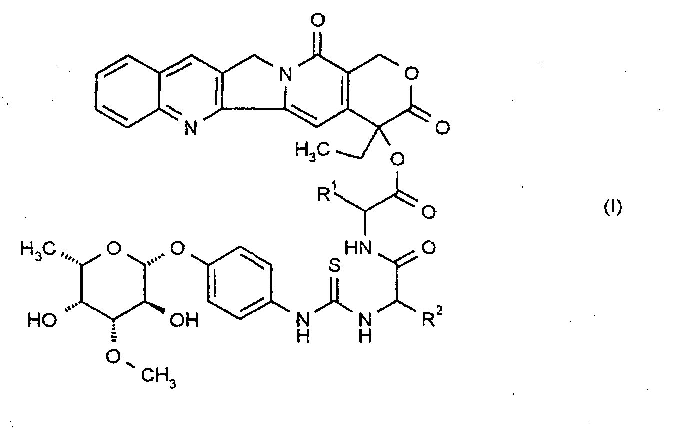 worin
- R¹: fur eine sterisch anspuchsvolle unpolare Seitenkette einer Aminosäure steht und
- R²: für eine basische Seitenkette einer Aminosäure steht
sowie deren Salze, Stereoisomere und Stereoisomerengemische.

Bevorzugt sind Verbindungen der Formel (I),
worin
- R¹: für einen verzweigten Alkylrest mit bis zu 4 Kohlenstoffatomen steht und
- R²: für einen Rest der Formel -(CH₂)ₙ-R³ steht, wobei
R3 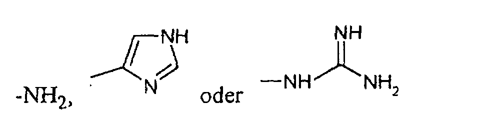 bedeutet und
n für eine Zahl 1 bis 4 steht

Besonders bevorzugt sind Verbindungen der allgemeinen Formel (I), worin
- R¹: für einen verzweigten Alkylrest der Formeln 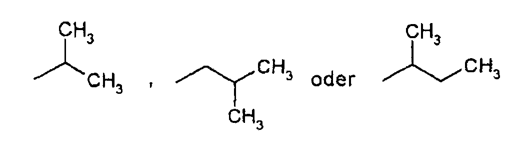 steht und
- R²: für einen Rest der Formeln 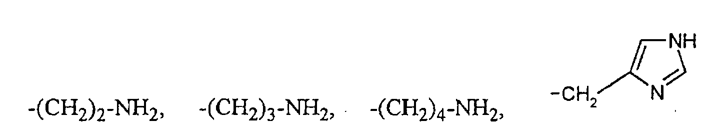 oder

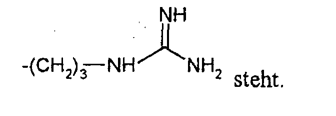

Der Camptothecin-Baustein kann in der 20(R)- oder in der 20(S)-Konfiguration oder als Gemisch dieser beiden steroisomeren Formen vorliegen. Bevorzugt ist die 20(S)-Konfiguration.

Die Aminosäuren können in der L- oder in der D-Konfiguration auftreten oder auch als Gemisch von D- und L-Form.

Der Begriff "Aminosäuren" bezeichnet insbesondere die in der Natur vorkommenden α-Aminosäuren, umfaßt darüber hinaus aber auch deren Homologe, Isomere und Derivate. Als Beispiel für Isomere können Enantiomere genannt werden. Derivate können beispielsweise mit Schutzgruppen versehene Aminosäuren sein.

Unter Aminosäuren mit "sterisch anspruchsvollen" Seitenketten werden solche Aminosäuren verstanden, deren Seitenkette in der β- oder γ-Position eine Verzweigung aufweist; als Beispiele seien Valin und Isoleucin bzw. Leucin genannt.

Als typische Beispiele fur Aminosäuren mit unpolaren Seitenketten seien genannt: Alanin, Valin, Leucin, Isoleucin, Prolin, Tryptophan, Phenylalanin, Methionin.

Als typische Beispiele für Aminosäure mit basischen Seitenketten seien genannt: Lysin, Arginin, Histidin, Ornithin, Diaminobuttersäure.

Die erfindungsgemäßen Verbindungen liegen bevorzugt in Form ihrer Salze vor. Im allgemeinen seien hier Salze mit organischen oder anorganischen Säuren genannt.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Trifluoressigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B p-Toluolsulfonsäure, 1,5-Naphthalindisulfonsäure oder Camphersulfonsäure.

Die erfindungsgemäßen Glycokonjugate können beispielsweise hergestellt werden durch Verknüpfung von 20(S)-Camptothecin mit aktivierten Carboxylkomponenten, die ihrerseits Teile von geschützten Aminosäuren, Peptiden oder kohlenhydratmodifizierten Peptiden darstellen können.

Bevorzugt erfolgt der Aufbau des Glycokonjugats sequentiell, beginnend mit der Acylierung von 20(S)-Camptothecin mit einem N-geschützten carboxyl-aktivierten Baustein einer unpolaren sterisch anspruchsvollen Aminosäure in einem geeigneten Lösungsmittel, gegebenenfalls in Gegenwart einer Base, nach üblichen Methoden. Anschließend wird mittels bekannter Methoden selektiv die Aminoschutzgruppe abgespalten. Dann wird ein falls notwendig geeignet geschützter Baustein einer basischen Aminosäure angeknüpft und anschließend gegebenenfalls unter Erhalt der Seitenkettenschutzgruppe an der α-Aminofunktion deblockiert. Im Schlüsselschritt erfolgt die Verknüpfung mit dem Kohlenhydratrest durch Überführung von p-Aminophenyl-3-O-methyl-β-L-fucopyranosid in das entsprechende Isothiocyanat und anschließende Verknüpfung mit der deblockierten α-Aminogruppe des Peptidyl-Camptothecins. Gegebenenfalls noch vorhandene Seitenkettenschutzgruppen werden abgelöst und die freie Aminogruppe wird gegebenenfalls in ein geeignetes Ammoniumsalz überführt.

Die Erfindung betrifft somit weiterhin ein Verfahren zur Herstellung der Glycokonjugate der Formel (I) 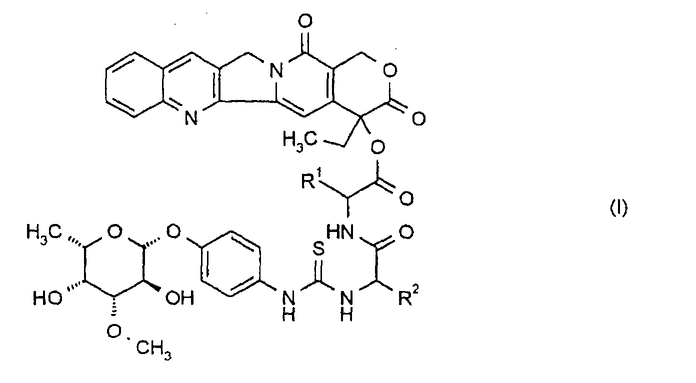 worin
- R¹: für eine sterisch anspruchsvolle unpolare Seitenkette einer Aminosäure steht und
- R²: für eine basische Seitenkette einer Aminosäure steht,
oder von deren Salzen, dadurch gekennzeichnet, daß man das Isothiocyanat der Formel (II) 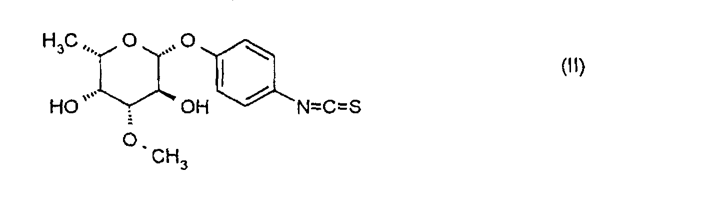 mit dem gegebenenfalls in der Seitenkette eine Schutzgruppe tragenden Peptidyl-Camptothecin der Formel (III) 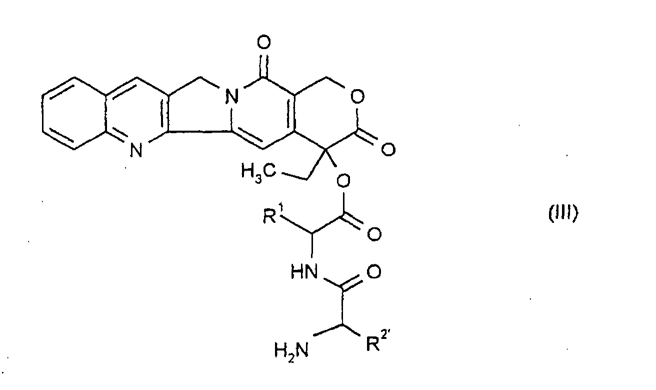 worin
- R¹: die obengenannte Bedeutung hat und
- R^{2'}: die Bedeutung des obengenannten basischen Rests R² hat, der darüberhinaus an der basischen Gruppe eine in der Peptidchemie übliche Schutzgruppe tragen kann
zu dem Glycokonjugat der Formel (IV) 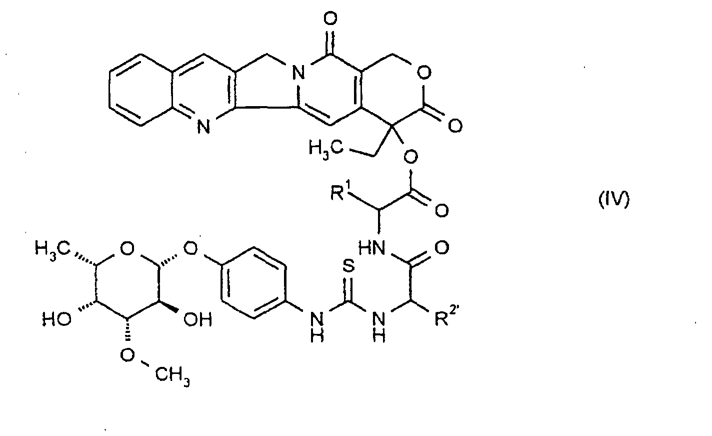 worin
- R¹ und R^{2'}: die oben angegebenen Bedeutungen haben,
umsetzt,
die gegebenenfalls vorhandene Seitenketten-Aminoschutzgruppe nach üblichen Methoden abspaltet und die erhaltene Verbindung gegebenenfalls in das gewünschte Salz überführt.

Denkbar ist auch eine andere Abfolge der Reaktionsschritte beim Aufbau der Zielverbindung. So kann gemäß einer ebenfalls bevorzugten Variante das p-Isothiocyanatophenyl-3-O-methyl-β-L-fucosid auch zuerst mit der gegebenenfalls geeignet geschützten terminalen basischen Aminosäure verknüpft werden, und dieser Baustein anschließend mit der freien Aminogruppe des Aminosäurekonjugats aus 20(S)-Camptothecin und der unpolaren, sterisch anspruchsvollen Aminosäure umgesetzt werden. Gegebenenfalls vorhandene Seitenkettenschutzgruppen werden abgelöst und die freie Aminogruppe gegebenenfalls in ein geeignetes Ammoniumsalz überführt

Die Erfindung betrifft daher weiterhin ein alternatives Verfahren zur Herstellung von Verbindungen der allgemeinen Formel (I) oder von deren Salzen, dadurch gekennzeichnet, daß man das Isothiocyanat der Formel (II) 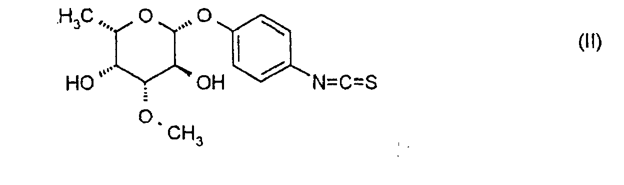 mit einer gegebenenfalls geeignet geschützten terminalen basischen Aminosäure der Formel (V) 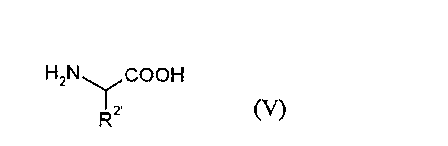 worin R^{2'} für eine basische Seitenkette einer Aminosäure steht, deren basische Gruppe geschützt sein kann,
zu einem Aminosäurekonjugat der Formel (VI) 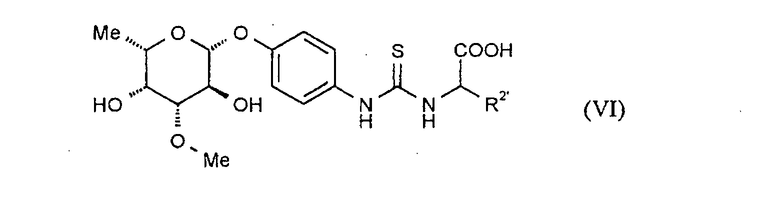 worin R^{2'} die vorstehend angegebene Bedeutung hat, umsetzt,
diese dann mit Aminosäurekonjugaten der Formel (VII) 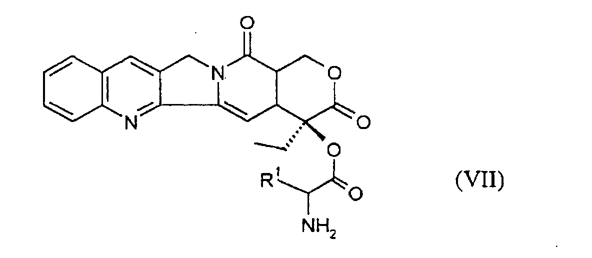 worin R¹ die vorstehend angegebene Bedeutung hat,
umsetzt,
die Seitenkettenschutzgruppe abspaltet und die Verbindungen gegebenenfalls in ein geeignetes Salz überführt.

Insbesondere nach Anknüpfung der ersten Aminosäure an Camptothecin können Diastereomerengemische entstehen. Reine Diastereomere der erfindungsgemäßen Verbindungen lassen sich nach den oben angegebenen Verfahren beispielsweise herstellen, in dem man nach Anknüpfung des ersten Aminosäurebausteins an das Camptothecin und anschließender Schutzgruppenabspaltung die Diastereomere in geeigneter Weise trennt. Aus einer diastereomerenreinen Zwischenverbindung kann auf dem oben angegebenen Weg die diastereomerenreine Zielverbindung hergestellt werden.

Es kann auch das Diastereomerengemisch der Zielverbindung in üblicher Weise in die einzelnen Diasteromere aufgetrennt werden.

Die Reaktionen können bei verschiedenen Druck- und Temperaturverhältnissen, beispielsweise 0,5 bis 2 bar, bzw. -30 bis +100°C, in geeigneten Lösungsmitteln wie Dimethylformamid (DMF), Tetrahydrofuran (THF), Dichlormethan, Chloroform, niederen Alkoholen, Acetonitril, Dioxan, Wasser oder in Gemischen der genannten Lösungsmittel durchgeführt werden. In der Regel sind Reaktionen in DMF, Dichlormethan oder THF/Dichlormethan bei Raumtemperatur und Normaldruck bevorzugt.

Für die Aktivierung der Carboxylgruppen kommen die in der Peptidchemie bekannten Kupplungsreagenzien wie sie z.B. in Jakubke/Jeschkeit: Aminosäuren, Peptide, Proteine; Verlag Chemie 1982 oder Tetrahedr. Lett. 34, 6705 (1993) beschrieben sind, in Frage. Bevorzugt sind beispielsweise N-Carbonsäureanhydride, Säurechloride oder gemischte Anhydride.

Weiterhin geeignet zur Aktivierung der Carboxylgruppen ist die Bildung von Addukten mit Carbodiimiden z.B. N,N'-Diethyl-, N,N'-Diisopropyl-, N,N'-Dicyclohexylcarbodiimid, N-(3-Dimethylaminopropyl)-N'-ethyl-carbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Chlorameisensäureisobutylester, oder Benzotriazolyloxy-tris-(dimethylamino)-phosphonium-hexafluorophosphat, 1-Hydroxybenzotriazol- oder N-Hydroxysuccinimidester. Weiterhin kann die Aminosäurekomponente auch in Form eines Leuchs'schen Anhydrids eingesetzt werden.

Als Basen können beispielsweise Triethylamin, Ethyl-diisopropylamin, Pyridin, N,N-Dimethylaminopyridin oder andere eingesetzt werden.

Als Schutzgruppen für Drittfunktionen der Aminosäuren können die in der Peptidchemie bekannten Schutzgruppen beispielsweise vom Urethan-, Alkyl-, Acyl-, Esteroder Amid-Typ eingesetzt werden.

Aminoschutzgruppen im Rahmen der Erfindung sind die üblichen in der PeptidChemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, tert-Butoxycarbonyl (Boc), Allyloxycarbonyl, Vinyloxycarbonyl, 3,4,5-Trimethoxybenzyloxycarbonyl, Phthaloyl, 2,2,2-Trichlorethoxycarbonyl, 2,2,2-Trichlor-tert-butoxycarbonyl, Menthyloxycarbonyl, 4-Nitrophenoxycarbony), Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl. Besonders bevorzugt sind die Fmoc-Gruppe und die Boc-Gruppe.

Bevorzugte Carboxylschutzgruppen sind lineare oder verzweigte C₁- bis C₄-Alkylester.

Die Abspaltung von Schutzgruppen in entsprechenden Reaktionsschritten kann zum Beispiel durch Säure- oder Base-Einwirkung, hydrogenolytisch oder auf andere Weise reduktiv erfolgen.

Die erfindungsgemäßen Glycokonjugate weisen sowohl in-vitro als auch in-vivo eine überraschend starke Antitumor-Wirksamkeit gegenüber verschiedenen Tumoren, insbesondere Lungen-, Brust-, Pankreas-, Melanom- und Dickdarm-Tumoren, verbunden mit einer großen Selektivität gegenüber nicht-malignen Zellen auf.

Sie eignen sich daher zur Behandlung von Krebserkrankungen und zwar sowohl in der Human- als auch in der Veterinärmedizin.

Zur vorliegenden Erfindung gehören pharmazeutische Zubereitungen, die neben nichttoxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer der erfindungsgemaßen Verbindung auch weitere pharmazeutische Wirkstoffe enthalten.

Im allgemeinen hat es sich sowohl in der Human- als auch in der Veterinärmedizin als vorteilhaft erwiesen, den erfindungsgemäßen Wirkstoff in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

### Biologische Testung

### 1. Wachstumsinhibitionstest zur Bestimmung der cytotoxischen Eigenschaften:

Die humanen Dickdarmzellinien SW 480 und HT 29 (ATCC-Nr. CCL 228 und HBT 38) sowie die Maus-Melanom-Zellinie B16F10 (CRL 6475) wurden in Rouxschalen in RPMI 1640 Medium unter Zusatz von 10 % FCS gezogen. Anschließend wurde trypsiniert und in RPMI plus 10 % FCS zu einer Zellzahl von 50.000 Zellen/ml für SW 480 und HT 29 und 20.000 Zellen für B16F10 aufgenommen. 100 µl Zellsuspension/Well wurden in eine 96 Mikrowellplatte gegeben und 1 Tag bei 37°C im CO₂-Brutschrank inkubiert. Anschließend wurden weitere 100 µl RPMI Medium und 1 µl DMSO mit den Prüfsubstanzen zugesetzt. Das Wachstum wurde nach Tag 6 überprüft. Dazu wurde zu jedem Mikrowell 25 µl MTT-Lösung (3-(4,5-Dimethylthiazol-2-yl)--2,5-diphenyltetrazolinbromid) mit einer Ausgangskonzentration von 5 mg/ml H₂O zugesetzt. Es wurde 5 Stunden im CO₂-Brutschrank bei 37°C inkubiert Anschließend wurde das Medium abgesaugt und 100 µl i-Propanol/Well zugesetzt. Nach 30 min Schütteln mit 100 µl H₂O wurde die Extinktion bei 595 nm mit einem Multiplate Reader (Bio..) 3550-UV gemessen.

Die cytotoxische Wirkung ist in der Tabelle 1 als IC₅₀-Wert jeweils für die SW 480- und HT 29- und B16F10-Zellinie angegeben:

**Tabelle 1:**

| Beispiel | IC₅₀ / nM SW 480 | IC₅₀ / nM HT 29 | IC₅₀ / nM B16F10 |
|---|---|---|---|
| 20(S)-Camptothecin | 10 | 5 | 20 |
| Beispiel 1 | 70 | 40 | 200 |
| Beispiel 2 | 100 | 40 | 300 |
| Beispiel 3 | 100 | 20 | 500 |
| Beispiel 4 | 100 | 20 | 500 |
| Beispiel 5 | 80 | 50 | 200 |
| Beispiel 6 | 60 | 30 | 300 |
| Beispiel 7 | 80 | 30 | 20 |
| Beispiel 8 | 200 | 100 | 800 |
| Beispiel 9 | 200 | 70 | 400 |
| Beispiel 10 | 100 | 50 | 300 |

### 2. Hämatopoetische Aktivität des Glycokonjugats im Vergleich zum zugrundeliegenden Wirkstoff:

### Material und Methoden:

Knochenmarkzellen wurden aus dem Femur der Maus gespült. 10⁵-Zellen wurden in McCoy 5A-Medium (0,3 % Agar) zusammen mit rekombinanten murinen GM-CSF (Genzyme; Stammzellen-Kolonienbildung) und den Substanzen (10⁻⁴ bis 100 µg/ml) bei 37°C und 7 % CO₂ inkubiert. 7 Tage später wurden die Kolonien (<50 Zellen) und Kluster (17-50 Zellen) ausgezählt.

### Ergebnisse:

Wie in Tab. 2 dargestellt, zeigen die untersuchten Glycokonjugate eine gegenüber dem zugrundeliegenden Wirkstoff drastisch verminderte Hemmung der Knochenmarkstammzellproliferation.

**Tabelle 2:**

| Hemmung der CSF-induzierten Proliferation von Knochenmarkstammzellen der Maus | |
|---|---|
| | IC₅₀ [ng/ml] |
| 20(S)-Camptothecin | 0,05 |
| Beispiel 1 | 15 |
| Beispiel 2 | 30 |
| Beispiel 3 | 15 |
| Beispiel 4 | 15 |
| Beispiel 5 | 20 |
| Beispiel 6 | 10 |
| Beispiel 7 | 8 |
| Beispiel 8 | 45 |
| Beispiel 9 | 15 |
| Beispiel 10 | 30 |

### 3. In-vivo-Hemmung des Tumorwachstums im Nacktmaus-Modell

### Material:

Für alle in-vivo-Experimente zur Untersuchung der Hemmung des Tumorwachstums wurden athymische Nacktmäuse (NMRI nu/nu-Stamm) verwendet. Das ausgewählte großzellige Lungenkarzinom LXFL 529 wurde durch serielle Passage in Nacktmäusen entwickelt. Der menschliche Ursprung des Tumors wurde durch isoenzymatische und immunohistochemische Methoden belegt.

### Experimenteller Aufbau:

Der Tumor wurde subcutan in beide Flanken von 6 bis 8 Wochen alten nu/nu-Nacktmäusen implantiert. Die Behandlung wurde abhängig von der Verdopplungszeit gestartet, sobald die Tumoren einen Durchmesser von 5-7 mm erreicht hatten. Die Mäuse wurden der Behandlungsgruppe und der Kontrollgruppe (5 Mäuse pro Gruppe mit 8 - 10 auswertbaren Tumoren) durch Randomisieren zugeteilt. Die einzelnen Tumoren der Kontrollgruppe wuchsen alle progressiv.

Die Größe der Tumoren wurde in zwei Dimensionen mittels Schieblehre vermessen. Das Tumorvolumen, das gut mit der Zellzahl korreliert, wurde anschließend für alle Auswertungen verwendet. Das Volumen wurde gemäß der Formel "Länge x Breite x Breite / 2" berechnet ([a x b²] / 2, a bzw. b stehen für zwei rechtwinklig angeordnete Durchmesser).

Die Werte des relativen Tumorvolumens (RTV) wurden für jeden einzelnen Tumor durch Dividieren der Tumorgröße am Tag X mit der Tumorgröße des Tages 0 (zum Zeitpunkt der Randomisierung) berechnet. Die mittleren Werte des RTV wurden dann für die weitere Auswertung verwendet.

Die Hemmung des Tumorvolumens (relatives Tumorvolumen der Testgruppe/Kontrollgruppe x 100, T/C in %) war der abschließende Meßwert.

### Behandlung:

Die Applikation der Verbindungen erfolgte beispielsweise an Tag 0, 1 und 2 nach Randomisierung intravenös (i.v.), wobei die Gesamtdosis pro Tag über 2 Gaben gesplittet wurde.

### Ergebnisse:

Die therapeutische Wirksamkeit der erfindungsgemäßen Glycokonjugate aus Beispiel 1 und 2 ist beispielhaft am großzelligen humanen Lungentumorxenograft LXFL 529 dargestellt. Die Therapie führt bei der maximal tolerablen Dosis (MTD) und bei 1/2 MTD zur kompletten bis deutlichen Tumorremission. Auch an anderen Tumoren kann eine exzellente Wirkung demonstriert werden.

**Tabelle 3:**

| Therapie | Dosis [mg/kg/Tag] | Überlebenszeit [Tage] | | Zahl der Tumoren | opt. T/C [%] | relatives Körpergewicht an Tag 7 [% des Tages 0] |
|---|---|---|---|---|---|---|
| Kontrolle | - | 7 | >28 | 8 | 100 | 104 |
| | | >28 | >28 | | | |
| | | >28 | | | | |
| Beispiel 1 | 16 | >28 | >28 | 10 | 0 | 95 |
| | | >28 | >28 | | (Tag 28) | |
| | | >28 | | | | |
| Beispiel 1 | 8 | 0 | >28 | 8 | 0 | 95 |
| | | >28 | >28 | | (Tag 14) | |
| | | >28 | | | | |
| Beispiel 2 | 32 | >28 | >28 | 8 | 0 | 98 |
| | | >28 | | 20 | (Tag 21) | |
| | | 1 | | | | |
| Beispiel 2 | 16 | >28 | >28 | 8 | 3,3 | 103 |
| | | >28 | >28 | | (Tag 28) | |
| | | >28 | | | | |

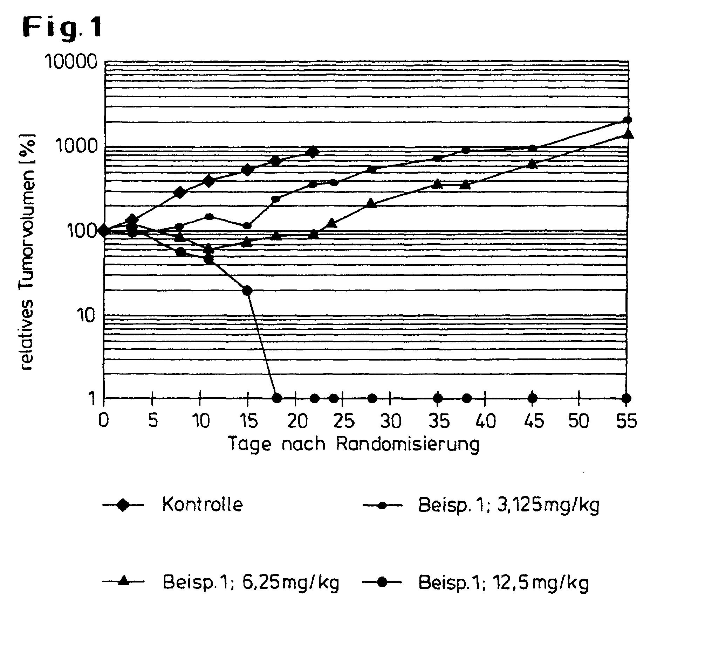

Die langanhaltende Komplettremission der Verbindung aus Beispiel 1 im Dosisbereich von 16 bis 8 mg/kg und die Dosisabhangigkeit der Wirkung ist in einem weiteren Experiment mit dem Therapieschedule Tag 1-3 i.v. in Fig. 1 dargestellt

### 4. Hydrolytische Stabilität:

Die erfindungsgemäßen Verbindungen aus Beispiel 1, 2, 8 und 9 werden in Wasser gelöst und zeigen nach 24h Stehen bei Raumtemperatur im HPLC deulich unter 1% Camptothecin-Freisetzung nach Flächenprozent.

Bei Lösen von 10µM der Verbindungen aus Beispiel 1 und 2 in RPMI-Medium plus 10% FCS und in 30 %igem humanen Vollblut in PBS Puffer erfolgte nach 24 h Stehen nur eine Camptothecin-Freisetzung unter 5 %

### Methode:

HPLC System Hewlett Packard HP 1050
Säule: Nucleosil 120-5 C 18 250 mm x 4 mm (Macherey & Nagel; Germany)
Eluent:
A: 0,01m KH₂PO₄ in H₂O (H₂= Milli-Pore grade)
B: 80% Acetonitril / 20% Eluent A
Fluß: 1,2ml
Gradient:
t₀: 20%B - t₄₀: 100%B
t₄₅: 100%B - t₄₇: 20%B
Detektion: 240 nm oder 370 nm

### 5. Lacton-Stabilisierung:

Die erfindungsgemäßen Glycokonjugate aus den Beispielen 1, 2, 8 und 9 werden in 80% Wasser und 20% Acetonitril gelöst und mit 2 Äquivalenten Natronlauge auf pH 9 eingestellt. Nach 1h Stehen bei Raumtemperatur liegt die Lactonring-Öffnung (Detektion nach obiger Methode) unter 5%.

### Beispiele

### Kohlenhydrat-Edukt:

### I) p-Aminophenyl-3-O-methyl-β-L-fucopyranosid:

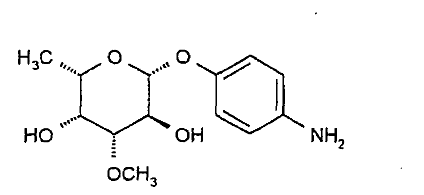

### I.a) p-Nitrophenyl-3-O-methyl-β-L-fucopyranosid:

6 g (21 mmol) p-Nitrophenyl-β-L-fucopyranosid in 300 ml absol. Methanol werden mit 7,84 g (31,5 mmol) Dibutylzinnoxid versetzt und 2 h unter Rückfluß erhitzt. Anschließend wird eingeengt, der Rückstand getrocknet und dann in 300 ml DMF aufgenommen. Nach Zugabe von 15,7 ml Methyliodid wird der Ansatz 40 h bei 70°C gerührt. Das Lösungsmittel wird im Vakuum entfernt und der Rückstand in 300 ml Dichlormethan aufgenommen Die Suspension wird filtriert, die verbleibende Lösung erneut eingeengt und einer Flash-Chromatographie (Dichlormethan/Methanol 99:1) unterzogen. Nach Einengen erhält man 3,82 g (61%) des Zielproduktes.

### I) p-Aminophenyl-3-O-methyl-β-L-fucopyranosid:

3,81 g (12,73 mmol) p-Nitrophenyl-3-O-methyl-β-L-fucopyranosid werden in Methanol gelöst und nach Zusatz von Platindioxid in einer Wasserstoffatmosphäre bei geringem Überdruck hydriert. Nach Abfiltrieren des Katalysators und Fällen mit Ether erhält man 3 g (88 %) des Zielprodukts. [DC: Dichlormethan/Methanol 9.1 R_{f}= 0,53].

### Peptidyl-Camptothecin-Edukte:

### II) 20(S)-20-O-{N^{ε}-[Fluorenyl-9-methoxycarbonyl]-L-lysyl-L/D-leucyl}camptothecin, Trifluoracetat:

### II) L 20(S)-20-O-{N^{ε}-[Fluorenyl-9-methoxycarbonyl]-L-lysyl-L-leucyl}camptothecin, Trifluoracetat:

### II) D 20(S)-20-O-{N^{ε}-[Fluorenyl-9-methoxycarbonyl]-L-lysyl-D-leucyl}camptothecin, Trifluoracetat:

### II.a) 20(S)-20-O-[N-(tert-Butoxycarbonyl)-L/D-leucyl]-camptothecin:

Eine Suspension von 10 g (28,7 mmol) 20(S)-Camptothecin in 250 ml absolutem Dimethylformamid wird unter Rühren mit 11,1 g (43 mmol) N-(tert-Butoxycarbonyl)-leucin-N-carbonsäureanhydrid sowie 1 g 4-(N,N-Dimethylamino)-pyridin versetzt. Nach 16 h Behandlung im Ultraschallbad bei Raumtemperatur setzt man weitere 3,7 g N-(tert-Butoxycarbonyl)-leucin-N-carbonsäureanhydrid zu und beläßt weitere 2h bei Raumtemperatur. Anschließend wird von restlichem nicht gelösten Camptothecin abgetrennt und das Filtrat im Vakuum eingeengt. Der Rückstand wird durch Flashchromatographie [Petrolether/Ethylacetat 1:1 -> 1:4] gereinigt. Man erhält 13,55 g (84 %) der Zielverbindung IIa). [DC. Acetonitril R_{f}= 0,47].

### II.b) 20(S)-20-O-L/D-Leucyl-camptothecin, Trifluoracetat:

### II. b) L 20(S)-20-O-L-Leucyl-camptothecin, Trifluoracetat

### II. b) D 20(S)-20-O-D-Leucyl-camptothecin, Trifluoracetat:

Eine Lösung von Verbindung II.a (13,55 g, 24,1 mmol) in einer Mischung aus 100 ml Dichlormethan und 40 ml wasserfreier Trifluoressigsäure wird für 30 min. bei Raumtemperatur gerührt. Nach Einengen im Vakuum auf ein kleines Volumen wird das Produkt mit Diethylether ausgefällt und gründlich mit Diethylether gewaschen. Im Dünnschichtchromatogramm (Acetonitril/Wasser 20:1) erkennt man einen Doppelfleck mit R_{f}-Werten von 0,4 und 0,32 [Ausb.: 9,5 g (68 %)].
Durch mehrmaliges Fällen aus Dichlormethan/Methanol mit Diethylether kann das Gemisch in beide Einzelkomponenten II.b) L und II.b) D aufgetrennt werden. Beide Formen erweisen sich als Diastereomere, die geringfügig unterschiedliche ¹H-NMR-Spektren ergeben:

| polares Diastereomer: | |
|---|---|
| 400-MHz-¹H-NMR (CD₂Cl₂/CD₃OD 1:1): δ | s C-H (B-Ring) 8,63 ppm |
| | s C-H (D-Ring) 7,4 ppm |

| unpolares Diastereomer: | |
|---|---|
| 400-MHz-¹H-NMR (CD₂Cl₂/CD₃OD 1:1): δ | s C-H (B-Ring) 8;60 ppm |
| | s C-H (D-Ring) 7,32 ppm |
| | |

In die weiteren Stufen können sowohl das Gemisch der beiden Formen als auch die aufgereinigten diastereomerenreinen Formen eingesetzt werden.

### II.c) 20(S)-20-O-[N^{α}-(tert-Butoxycarbonyl)-N^{ε}-(fluorenyl-9-methoxycarbonyl)-L-lysyl-L/D-leucyl]-camptothecin:

### II.c) L 20(S)-20-O-[N^{α}-(tert-Butoxycarbonyl)-N^{ε}-(fluorenyl-9-methoxycarbonyl)-L-lysyl-L-leucyl]-camptothecin:

### II.c) D 20(S)-20-O-[N^{α}-(tert-Butoxycarbonyl)-N^{ε}-(fluorenyl-9-methoxycarbonyl)-L-lysyl-D-leucyl]-camptothecin:

25,6 g (54,6 mmol) N^{α}-(tert-Butoxycarbonyl)-N^{ε}-(fluorenyl-9-methoxycarbonyl)-L-lysin und 11,1g (82 mmol) 1-Hydroxy-1H-benzotriazol Hydrat werden in 500 ml Dimethylformamid gelöst. Nach Zugabe von 12,6 g (1,2 Äq.) N-Ethyl-N'-(dimethylaminopropyl)-carbodiimid Hydrochlorid rührt man für 1 h bei Raumtemperatur Anschließend setzt man Verbindung II.b (26,2 g, 0,83 Äq.) und 7,83 ml (1 Äq.) Ethyl-diisopropylamin zu und rührt den Ansatz für weitere 16 h bei Raumtemperatur. Nach Einengen im Vakuum und Reinigung durch Flashchromatographie [Petrolether/Ethylacetat 1:2 -> Ethylacetat] erhält man die Zielverbindung (43,5 g, 87%) [DC: Acetonitril R_{f}= 0,44].
Wie auch in den weiteren Beispielen kann der Ansatz völlig analog auch mit jeder der aufgereinigten diastereomerenreinen Formen in II.b durchgeführt werden.

### II) 20(S)-20-O-{N^{ε}-[Fluorenyl-9-methoxycarbonyl]-L-lysyl-L/D-leucyl}-camptothecin, Trifluoracetat:

### II) L 20(S)-20-O-{N^{ε}-[Fluorenyl-9-methoxycarbonyl]-L-lysyl-L-leucyl}-camptothecin, Trifluoracetat

### II) D 20(S)-20-O-{N^{ε}-[Fluorenyl-9-methoxycarbonyl]-L-lysyl-D-leucyl}-camptothecin, Trifluoracetat

Die Verbindung II.c (43,3 g, 47,5 mmol) wird in 150 ml Dichlormethan aufgenommen und mit 50 ml wasserfreier Trifluoressigsäure versetzt. Die resultierende Lösung wird für 1 h bei Raumtemperatur gerührt. Nach Einengen auf ein kleines Volumen im Vakuum wird das Produkt durch Zugabe von Diethylether ausgefällt. Man erhält 39,4 g (90 %) der Zielverbindung [DC: Acetonitril/Wasser 10:1 R_{f} = 0,35]

### III) 20(S)-20-O-(L-Histidyl-L/D-valyl)-camptothecin, Trifluoracetat:

### III.a) 20(S)-20-O-[N-(tert-Butoxycarbonyl)-L/D-valyl]-camptothecin:

Eine Suspension von 10 g (28,7 mmol) 20(S)-Camptothecin in 500 ml absolutem Dimethylformamid wird unter Rühren mit 21,5 g (3 Äquivalenten) N-(tert-Butoxycarbonyl)-valin-N-carbonsäureanhydrid sowie 1 g 4-(N,N-Dimethylamino)-pyridin versetzt. Nach 16 h Behandlung im Ultraschallbad bei Raumtemperatur wird von restlichem nicht gelösten Camptothecin abgetrennt und das Filtrat im Vakuum eingeengt. Der Rückstand wird durch Flashchromatographie [Petrolether/Ethylacetat 1.3 -> Ethylacetat] gereinigt. Man erhält 11,65 g (73 %) der Zielverbindung. [DC: Acetonitril R_{f} = 0,44]. Wird die gleiche Reaktion statt in Dimethylformamid in Dichlormethan unter Rückflußbedingungen durchgeführt, so wird die Entstehung des Diastereomers mit L-Konfiguration des Valin-Bausteins stärker begünstigt.

### III.b) 20(S)-20-O-L/D-Valyl-camptothecin, Trifluoracetat:

### III.b) L 20(S)-20-O-L-Valyl-camptothecin, Trifluoracetat

### III.b) D 20(S)-20-O-D-Valyl-camptothecin, Trifluoracetat

Eine Lösung von Verbindung III.a (11,65 g, 21 mmol) in einer Mischung aus 100 ml Dichlormethan und 100 ml wasserfreier Trifluoressigsäure wird für 1 h bei Raumtemperatur gerührt. Nach Einengen im Vakuum auf ein kleines Volumen wird das Produkt mit Diethylether ausgefällt und gründlich mit Diethylether gewaschen. Das Produkt wird nochmals aus Dichlormethan/Methanol mit Diethylether gefällt und als Gemisch von diastereomeren Formen isoliert. Ausb.: 10,9 g (92 %) [DC: Acetonitril/Wasser 20:1 R_{f} = 0,31 und 0,39]. Eine höhere Aufreinigung des unpolaren Diasteromers mit L-Konfiguration des Valin-Bausteins ist auf dieser Stufe durch Kristallisation aus Methanol möglich:
8g des erhaltenen Produkts mit angereichertem unpolarem Diastereomers werden in 80ml Methanol gelöst, auf 0°C abgekühlt und in 10ml Schritten mit Diethylether versetzt. Nach Zugabe von insgesamt 60 ml Diethylether wird. das ausgefallene Produkt abgesaugt und getrocknet. Man erhält 4,6g (58%) an reinem unpolarem Diasteromer mit L-Konfiguration des Valin-Bausteins. Eine Nachfällung der Mutterlauge mit Diethylether ergibt weitere 730mg (9%) einer Produktfraktion mit einem Diastereomerenverhältnis von 1:18.

In die weiteren Reaktionen kann sowohl das Diastereomerengemisch als auch das aufgereinigte unpolare oder das polare Diastereomer eingesetzt werden. Die Umsetzungen laufen völlig analog.

### III.c) 20(S)-20-O-[N-(tert-Butoxycarbonyl)-L-histidyl-L/D-valyl]-camptothecin:

### III.c) L 20(S)-20-O-[N-(tert-Butoxycarbonyl)-L-histidyl-L-valyl]-camptothecin

### III.c) D 20(S)-20-O-[N-(tert-Butoxycarbonyl)-L-histidyl-D-valyl]-camptothecin

5,95 g (23,3 mmol) N-(tert-Butoxycarbonyl)-L-histidin und 4,73 g 1-Hydroxy-1H benzotriazol Hydrat werden in 200 ml Dimethylformamid gelöst. Nach Zugabe von 5,38 g N-Ethyl-N'-(dimethylaminopropyl)-carbodiimid Hydrochlorid ruhrt man für 1 h bei Raumtemperatur. Anschließend setzt man Verbindung III.b (10,9 g, 19,44 mmol) und 6,7 ml Ethyl-diisopropylamin zu und rührt den Ansatz für weitere 16 h bei Raumtemperatur. Nach Einengen im Vakuum und Reinigung durch Flashchromatographie [Acetonitril/Wasser 50:1 -> 20:1] erhält man die Zielverbindung, [DC: Acetonitril/Wasser 10:1 R_{f} = 0,42] die sofort weiter umgesetzt wird.

### III) 20(S)-20-O-(L-Histidyl-L/D-valyl)-camptothecin, Bis-Trifluoracetat:

### III) L 20(S)-20-O-(L-Histidyl-L-valyl)-camptothecin, Bis-Trifluoracetat

### III) D 20(S)-20-O-(L-Histidyl-D-valyl)-camptothecin, Bis-Trifluoracetat

Die Verbindung III.c wird in 100 ml Dichlormethan aufgenommen und mit 50 ml wasserfreier Trifluoressigsäure versetzt. Die resultierende Lösung wird für 30 min bei Raumtemperatur gerührt. Nach Einengen auf ein kleines Volumen im Vakuum wird das Produkt durch Zugabe von Diethylether ausgefällt. Man erhält 13,05 g (83 %) der Zielverbindung [DC: Acetonitril/Wasser/Eisessig 5:1:0,2 R_{f} = 0,2].

### IV) 20(S)-20-O-{N^{ε}-[Fluorenyl-9-methoxycarbonyl]-D-lysyl-L/D-leucyl}-camptothecin, Trifluoracetat:

### IV) L 20(S)-20-O-{N^{ε}-[Fluorenyl-9-methoxycarbonyl]-D-lysyl-L-leucyl}-camptothecin, Trifluoracetat:

### IV) D 20(S)-20-O-{N^{ε}-[Fluorenyl-9-methoxycarbonyl]-D-lysyl-D-leucyl}-camptothecin, Trifluoracetat:

Die Synthese dieser Verbindung erfolgt in völliger Analogie zu der diastereomeren Verbindung II. In der Stufe II.c wird anstelle von N^{α}-(tert-Butoxycarbonyl)-N^{ε}-(fluorenyl-9-methoxycarbonyl)-L-lysin das entsprechende D-Isomer eingesetzt [DC: Acetonitril/Wasser 10:1 R_{f} = 0,4].

### V) 20(S)-20-O-{N^{ε}-[Fluorenyl-9-methoxycarbonyl]-L-ornithyl-L/D-leucyl}-camptothecin, Trifluoracetat:

### V) L 20(S)-20-O-{N^{ε}-[Fluorenyl-9-methoxycarbonyl]-L-ornithyl-L-leucyl}-camptothecin, Trifluoracetat

### V) D 20(S)-20-O-{N^{ε}-[Fluorenyl-9-methoxycarbonyl]-L-ornithyl-D-leucyl}-camptothecin, Trifluoracetat

Die Synthese dieser Verbindung erfolgt in völliger Analogie zu der entsprechenden Lysin-Verbindung II. In der Stufe II.c wird anstelle von N^{α}-(tert-Butoxycarbonyl)-N^{ε}-(fluorenyl-9-methoxycarbonyl)-L-lysin N^{α}-(tert-Butoxycarbonyl)-N^{ε}-(fluorenyl-9-methoxycarbonyl)-L-ornithin eingesetzt. [DC: Acetonitril/Wasser 20:1 R_{f} = 0,125].

### VI) 20(S)-20-O-(L-Arginyl-L/D-leucyl)-camptothecin, Tri-Trifluoracetat:

### VI) L 20(S)-20-O-{L-Arginyl-L-leucyl}-camptothecin, Tri-Trifluoracetat

### VI) D 20(S)-20-O-{L-Arginyl-D-leucyl}-camptothecin, Tri-Trifluoracetat

### VI.a) 20(S)-20-O-(Tri-tert-butoxycarbonyl-L-arginyl-L/D-leucyl)-camptothecin

### VI.a) L 20(S)-20-O-{Tri-tert-butoxycarbonyl-L-arginyl-L-leucyl}-camptothecin

### VI.a) D 20(S)-20-O-{Tri-tert-butoxycarbonyl-L-arginyl-D-leucyl}-camptothecin

200 mg (0,42 mmol) Tri-tert-butoxycarbonyl-L-arginin und 80 mg 1-Hydroxy-1H benzotriazol Hydrat werden in 20 ml Dimethylformamid gelöst. Nach Zugabe von 97 mg N-Ethyl-N'-(dimethylaminopropyl)-carbodiimid Hydrochlorid rührt man für 1 h bei Raumtemperatur. Anschließend setzt man Verbindung II.b (200 mg, 0,35 mmol) und 217 µl Ethyl-diisopropylamin zu und rührt den Ansatz für weitere 3 d bei Raumtemperatur. Nach Einengen im Vakuum und behandeln mit Wasser erhält man die Zielverbindung [DC: Acetonitril/Wasser 5:1:0,2 R_{f} = 0,77] die sofort weiter umgesetzt wird.

### VI) 20(S)-20-O-{L-Arginyl-L/D-leucyl}-camptothecin, Tri-Trifluoracetat

### VI) L 20(S)-20-O-{L-Arginyl-L-leucyl}-camptothecin, Tri-Trifluoracetat

### VI) D 20(S)-20-O-{L-Arginyl-D-leucyl}-camptothecin, Tri-Trifluoracetat

0,35 mmol der Verbindung aus VI.a) werden in 10 ml Dichlormethan mit 5 ml wasserfreier Trifluoressigsäure für 2 h bei Raumtemperatur gerührt. Man engt ein und fällt zweimal aus Dichlormethan/Methanol mit Diethylether um. Man erhält 280 mg (82%) der Zielverbindung [DC: Acetonitril/Wasser 10:3:1,5 R_{f} = 0,42].

### VII) L 20(S)-20-O-{N^{ε}-[Fluorenyl-9-methoxycarbonyl]-L-lysyl-L-valyl}-camptothecin, Trifluoracetat

### VII.a) L 20(S)-20-O-[N^{α}-(tert-butoxycarbonyl)-N^{ε}-(fluorenyl-9-methoxycarbonyl)-L-lysyl-L-valyl]-camptothecin

10,02 g (21,4 mmol) N -(tert-butoxycarbonyl)-N -(fluorenyl-9-methoxycarbonyl)-Llysin und 4,4g (32,1 mmol) 1-Hydroxy-1H-benzotriazol Hydrat werden in 400 ml Dimethylformamid gelöst. Nach Zugabe von 4,92 g (1,2Äq.) N-Ethyl-N'-(dimethylaminopropyl)-carbodiimid Hydrochlorid rührt man für 30 min bei 0°C. Anschließend setzt man 10g (17,8 mmol) des unpolaren Diastereomers III.b Lvon Verbindung III.b und 9,2 ml (3Äq.) Ethyl-diisopropylamin zu und rührt den Ansatz für weitere 16 h bei Raumtemperatur. Nach Einengen im Vakuum wird der Rückstand 30 min mit 500ml Wasser verrührt und abgesaugt. Das Produkt wird in 400 ml Dichlormethan aufgenommen, das restliche Wasser entfernt, die Lösung auf 200ml aufkonzentriert und dann mit 800 ml Diethylether gefällt. Man saugt ab und wäscht den Rückstand mit Diethylether. Man erhält 14,712 g der Zielverbindung (92 %) [DC: Acetonitril R_{f}= 0,6]

### VII) L 20(S)-20-O-{N-[Fluorenyl-9-methoxycarbonyl]-L-lysyl-L-valyl}-camptothecin, Trifluoracetat

Die Verbindung VII.a) L (14,65 g, 16,3 mmol) wird in 300 ml Dichlormethan aufgenommen und bei 0°C mit 50 ml wasserfreier Trifluoressigsäure versetzt. Die resultierende Lösung für 2 h unter Eiskühlung gerührt. Nach Einengen auf ein kleines Volumen im Vakuum wird das Produkt durch Zugabe von Diethylether ausgefällt. Man erhält 13,8g (93%) der Zielverbindung [DC: Acetonitril/Wasser 10:1 R_{f} = 0,35].

### Beispiel 1:

### 20(S)-20-O-{N^{α}-[O-(3-O-Methyl-β-L-fucopyranosyl)-4-hydroxy-phenylaminothiocarbonyl]-L-lysyl-L/D-leucyl}-camptothecin, Hydrochlorid:

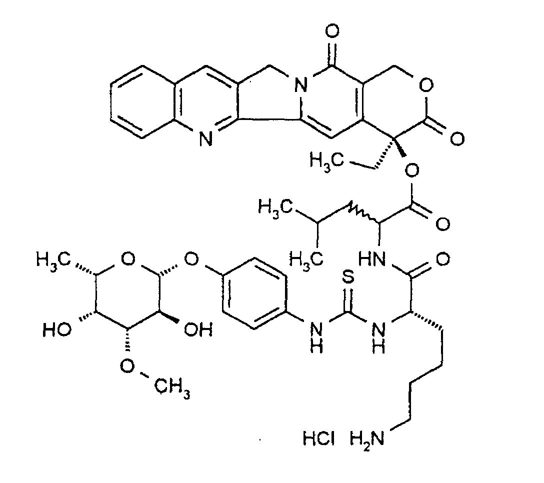

### 1.a) 20(S)-20-O-{N^{α}-[O-(3-O-Methyl-β-L-fucopyranosyl)-4-hydroxyphenylamino-thiocarbonyl]-N^{ε}-[fluorenyl-9-methoxycarbonyl]-L-lysyl-L/D-leucyl}-camptothecin:

Eine Lösung von 9,82 g (36,5 mmol) p-Aminophenyl-3-O-methyl-β-L-fucopyranosid (Beispiel I) in 500 ml Dioxan/Wasser 1:1 wird unter Rühren mit 3,94 ml Thiophosgen (1,4 Äq.) versetzt. Nach 10 min versetzt man mit 4 Äquivalenten Ethyldiisopropylamin, engt anschließend im Vakuum ein und trocknet den Rückstand für 1 h im Ölpumpenvakuum. Das erhaltene Isothiocyanat wird in 500 ml absolutem Dimethylformamid gelöst und mit 30,4 g (32,8 mmol) von Verbindung II sowie 22,6 ml Ethyl-diisopropylamin versetzt. Man rührt für 16 h bei Raumtemperatur, engt dann im Vakuum ein und verrührt mit Wasser Den Rückstand reinigt man durch Flash-Chromatographie [Acetonitril -> Acetonitril/Wasser 30:1]. Das Produkt wird aus Dichlormethan/Methanol mit Diethylether umgefällt und mit Diethylether gewaschen. Man erhält 28,7 g (78 %) des Zielproduktes [DC: Acetonitril/Wasser 10:1 R_{f}= 0,44].

### 1.b) 20-O-{N^{α}-[O-(3-O-Methyl-β-L-fucopyranosyl)-4-hydroxy-phenylaminothiocarbonyl]-L-lysyl-L/D-leucyl}-camptothecin:

28,6 g (25,5 mmol) des Konjugats 1.a) werden in 200 ml Dimethylformamid mit 5 ml Piperidin versetzt. Nach 2 h Rühren bei Raumtemperatur engt man ein und digeriert den Rückstand zweimal mit Dichlormethan und setzt Diethylether zu. Dann wird in Dimethylformamid/Dichlormethan aufgenommen und mit Ether gefällt. Dieser Reinigungsprozess wird zweimal wiederholt. Das Produkt wird abgesaugt und mit Ether gewaschen Ausb.: 19,5 g (85%) [DC: Acetonitril/Wasser/Eisessig 5:1:0,2 R_{f}= 0,3].

### 1) 20(S)-20-O-{N^{α}-[O-(3-O-Methyl-β-L-fucopyranosyl)-4-hydroxy-phenylamino-thiocarbonyl]-L-lysyl-L/D-leucyl}-camptothecin, Hydrochlorid:

10 g (11,1 mmol) der Verbindung aus Beispiel 1.b werden in 100 ml Wasser aufgenommen, mit 11,1 ml (1 Äq.) einer 1N HCl-Lösung versetzt und lyophilisiert. Anschließend wird das Produkt mehrmals aus Dichlormethan/Methanol mit Diethylether gefällt. Ausb.: 9,15 g (88%) [DC: Acetonitril/Wasser/Eisessig 5:1-0,2 R_{f} = 0,3].

### Beispiel 2:

### 20(S)-20-O-{N-[O-(3-O-Methyl-β-L-fucopyranosyl)-4-hydroxy-phenylaminothiocarbonyl]-L-histidyl-L/D-valyl}-camptothecin, Hydrochlorid:

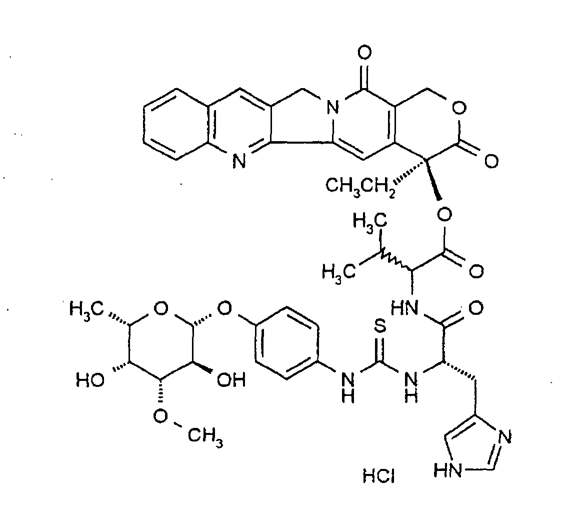

Eine Lösung von 7,14 g (26,5 mmol) p-Aminophenyl-3-O-methyl-β-L-fucopyranosid (Kohlenhydrat-Edukt I) in 300 ml Dioxan/Wasser 1:1 wird unter Rühren mit 2,86 ml Thiophosgen (1,4 Äq.) versetzt. Nach 10 min. versetzt man mit 4 Äquivalenten Ethyl-diisopropylamin, engt anschließend im Vakuum ein und trocknet den Rückstand für 1 h im Ölpumpenvakuum. Das erhaltene Isothiocyanat wird in 500 ml absolutem Dimethylformamid gelöst und mit 17,45 g (22 mmol) von Verbindung III sowie 22,7 ml Ethyl-diisopropylamin versetzt. Man rührt fur 16 h bei Raumtemperatur, engt dann im Vakuum ein und nimmt den Rückstand in Wasser auf. Man stellt mit 1N wäßriger Natronlauge auf pH 7,8 ein und saugt ab. Der Rückstand wird nach Trocknung im Hochvakuum zweimal aus Dichlormethan/Methanol mit Diethylether umgefällt und mit Diethylether gewaschen Man erhält 17,97 g (91 %) des Zielproduktes, das anschließend mit 1 Äquivalent 0,1 N wäßriger Salzsäure in das Hydrochlorid überführt wird [DC: Acetonitril/Wasser/Eisessig 5:1:0,2 R_{f} = 0,36] [FAB-MS: m/z = 896 (M + H⁺)].

### Beispiel 3:

### 20(S)-20-O-{N^{α}-[O-(3-O-Methyl-β-L-fucopyranosyl)-4-hydroxy-phenylaminothiocarbonyl]-D-lysyl-D-leucyl}-camptothecin, Hydrochlorid;

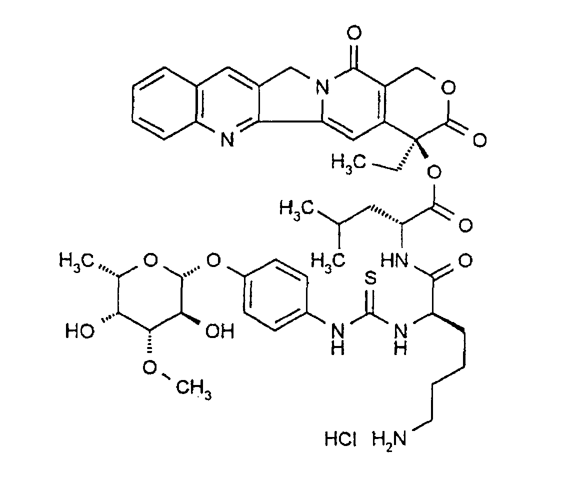

Die Synthese erfolgt völlig analog zu Beispiel 1. Hierbei wird von den Edukten I und IV.D ausgegangen, wobei ab der Stufe des 20-O-Leucyl-Camptothecins II.b das polare Diasteromer II.b) D eingesetzt wurde. [DC: Acetonitril/Wasser/Eisessig 5:1:0,2 R_{f} = 0,31] [FAB-MS: m/z = 901 = M + H⁺]

### Beispiel 4:

### 20(S)-20-O-{N^{α}-[O-(3-O-Methyl-β-L-Fucopyranosyl)-4-hydroxy-phenylaminothiocarbonyl]-L-ornithyl-D-leucyl}-camptothecin, Hydrochlorid;

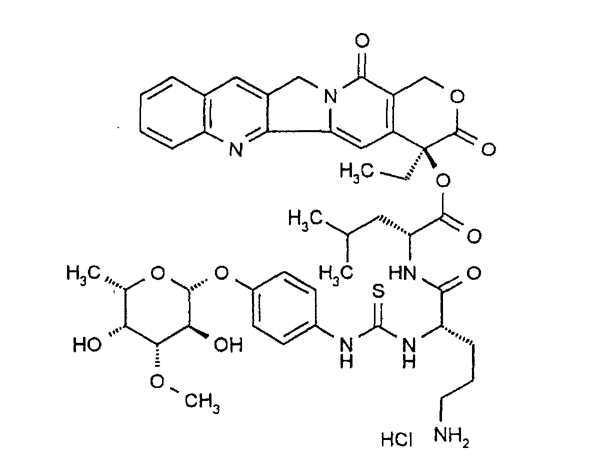

Die Synthese erfolgt völlig analog zu Beispiel 1. Hierbei wird von den Edukten I und V) D ausgegangen, wobei ab der Stufe des 20-O-Leucyl-Camptothecins II.b das polare Diasteromer II.b) D mit D-Leu-Konfiguration eingesetzt wurde. [DC: Acetonitril/Wasser/Eisessig 5:1:0,2 R_{f} = 0,25].

### Beispiel 5:

### 20(S)-20-O-{N^{α}-[O-(3-O-Methyl-β-L-fucopyranosyl)-4-hydroxy-phenylaminothiocarbonyl]-L-arginyl-D-leucyl}-camptothecin;

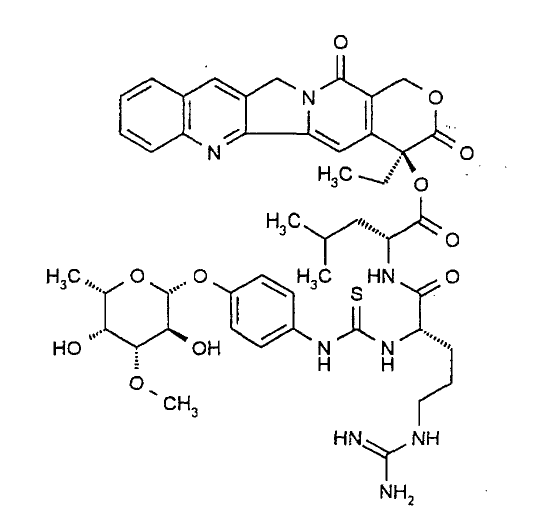

Eine Lösung von 73 mg (0,27 mmol) p-Aminophenyl-3-O-methyl-β-L-fucopyranosid (Edukt I) in 20 ml Dioxan/Wasser 1:1 wird unter Rühren mit 30 µl Thiophosgen (1,4 Äq.) versetzt. Nach 10 min. versetzt man mit 4 Äquivalenten Ethyldiisopropylamin, engt anschließend im Vakuum ein und trocknet den Rückstand für 1 h im Ölpumpenvakuum. Das erhaltene Isothiocyanat wird in 20 ml absolutem Dimethylformamid gelöst und mit 175 mg (0,18 mmol) von Verbindung VI) D sowie 620 µl Ethyl-diisopropylamin versetzt. Zur Synthese von Verbindung VI) D wird hier ab der Stufe des 20-O-Leucyl-Camptothecins II.b das polare Diastereomer II.b) D eingesetzt. Man rührt für 16 h bei Raumtemperatur, engt dann im Vakuum ein und verrührt mit Dichlormethan Der Rückstand wird anschließend aus Dichlormethan/Methanol mit Diethylether umgefällt und mit Diethylether gewaschen Man lyophilisiert anschließend aus Dioxan/Wasser und kristallisiert dann erneut aus Dichlormethan/Methanol mit Diethylether. Man erhält 154 mg (90 %) des Zielproduktes [DC: Acetonitril/Wasser/Eisessig 5.1:0,2 R_{f}= 0,39] [FAB-MS: m/z = 929 = M+H⁺].

### Beispiel 6:

### 24(S)-20-O-{N^{α}-[O-(3-O-Methyl-β-L-fucopyranosyl)-4-hydroxy-phenylaminothiocarbonyl]-L-lysyl-D-leucyl}-camptothecin, Hydrochlorid;

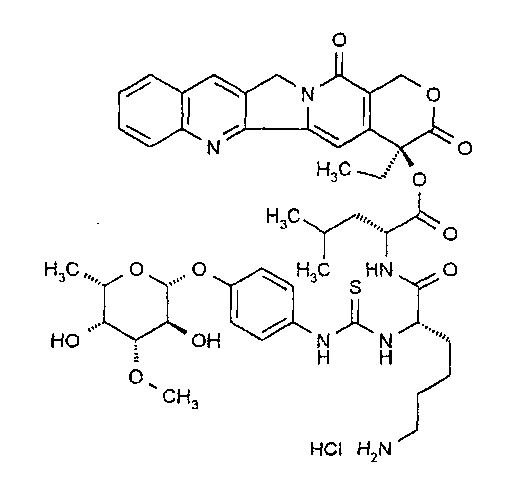

Die Verbindung wird in Analogie zu Beispiel 1 hergestellt, wobei auf der Stufe des 20-O-Leucyl-Camptothecins II.b das polare Diastereomer II.b) D mit D-Leucin-Konfiguration eingesetzt wurde.

Alternativ kann die Verbindung aus Beispiel 1 z.B. auch durch präparative HPLC in die einzelnen isomeren Formen aufgetrennt werden.

### Bedingungen:

Trennsäule: Macherey & Nagel 250 x 21 mm Nucleosil 100-7 C 18 AB
Laufmittel A: H₂O + 0,1M KH₂PO₄
Laufmittel B: Acetonitril / Wasser 4:1 + 0,02M KH₂PO₄
Fluß: 10 ml/min
Inj.-Volumen: 1500µl
Gradient: 0-30 % B
   4-30
   20-90
   22-90
   24-30
Wellenlänge: 215 nm

Nach der HPLC-Trennung werden die entsprechenden Fraktionen lyophilisiert und der Rückstand dann mehrmals aus Dichlormethart/Methanol mit Diethylether gefällt. Anschließend stellt man auf pH 8-9 ein, filtriert ab und überführt den Rückstand mit 0,1N Salzsäure in das Hydrochlorid.

Im ¹H-NMR-Spektrum zeigen die Isomeren in Beispiel 6 und 7 unterschiedliche chemische Verschiebungen insbesondere für die beiden Singuletts der aromatischen H-Atome im Camptothecin-B-Ring bzw. D-Ring.

| Diasteromer mit D-Leucin: | |
|---|---|
| 400-MHz-¹H-NMR (CD₂Cl₂/CD₃OD 1:1): δ | s C-H (B-Ring) 8,52 ppm |
| | s C-H (D-Ring) 7,42 ppm |
| [FAB-MS: m/z = 901 = M + H⁺] | |

### Beispiel 7:

### 20(S)-20-O-{N^{α}-[O-(3-O-Methyl-β-L-fucopyranosyl)-4-hydroxy-phenylaminothiocarbonyl]-L-lysyl-L-leucyl}-camptothecin, Hydrochlorid;

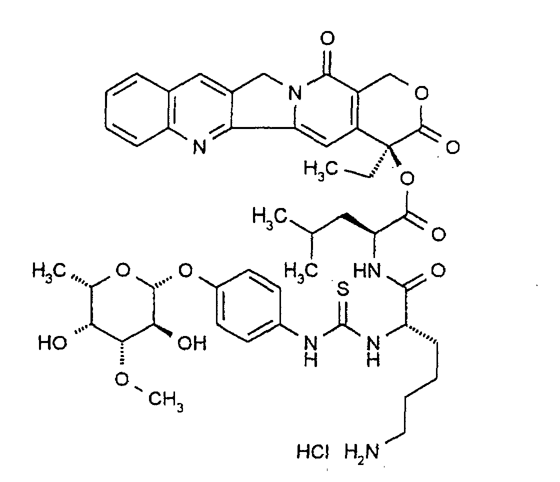

Die Verbindung wird in Analogie zu Beispiel 1 hergestellt, wobei auf der Stufe des 20-O-Leucyl-Camptothecins II.b das unpolare Diastereomer II.b) L mit L-Konfiguration des Leucins eingesetzt wurde.

Alternativ kann die Verbindung aus Beispiel 1 z.B. auch durch präparative HPLC in die einzelnen isomeren Formen aufgetrennt werden.

Bedingungen: wie in Beispiel 6 angegeben
Nach der HPLC-Trennung werden die entsprechenden Fraktionen lyophilisiert und der Rückstand dann mehrmals aus Dichlormethan/Methanol mit Diethylether gefällt. Anschließend stellt man auf pH 8-9 ein, filtriert ab und überführt den Rückstand mit 0,1 N Salzsäure in das Hydrochlorid.

Im ¹H-NMR-Spektrum zeigen die Isomeren in Beispiel 6 und 7 unterschiedliche chemische Verschiebungen insbesondere z.B für die beiden Singuletts der aromatischen H-Atome im Camptothecin-B-Ring bzw. D-Ring

| Diastereomer mit L-Leucin | |
|---|---|
| 400-MHz-¹H-NMR (CD₂Cl₂/CD₃OD 1:1): δ | s C-H (B-Ring) 8,6 ppm |
| | s C-H (D-Ring) 7,35 ppm |
| [FAB-MS: m/z = 901 = M + H⁺] | |

### Beispiel 8:

### 20(S)-20-O-{N^{α}-[O-(3-O-Methyl-β-L-fucopyranosyl)-4-hydroxy-phenylaminothiocarbonyl]-L-lysyl-L-valyl}-camptothecin, Hydrochlorid;

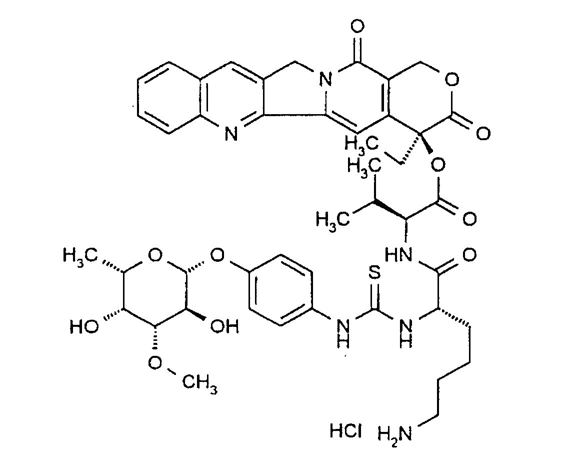

Die Verbindung wird in der unpolaren Serie ausgehend von der Verbindung VII) L in Analogie zu Beispiel 1 hergestellt.
Das Diastereomerenverhältnis kann durch analytische HPLC ermittelt werden. Gegebenenfalls durch Kristallisation aus Methanol kann eine weitere Aufreinigung des Diastereomers mit L-Konfiguration des Valins erreicht werden (>20:1).

HPLC-Bedingungen:
Trennsäule: Macherey & Nagel 250 x 4 mm Nucleosil 100-7 C18 AB
Laufmittel A: H₂O + 0,1M KH₂PO₄
Laufmittel B: Acetonitril / Wasser 4:1 + 0,02M KH₂PO₄
Fluß: 1 ml/min
Inj. -Volumen: 15 µl

Im ¹H-NMR-Spektrum zeigt das reine Diastereomer nur einen Signalsatz z.B. für die beiden Singuletts der aromatischen H-Atome im Camptothecin-B-Ring bzw. D-Ring.

| | |
|---|---|
| 400-MHz-¹H-NMR (CD₂Cl₂/CD₃OD 1:1): δ | s C-H (B-Ring) 8,55 ppm |
| | s C-H (D-Ring) 7,35 ppm |
| [FAB-MS m/z = 887 = M + H⁺] | |

Eine alternative Vorgehensweise beim Aufbau der Verbindung aus Beispiel 8 ist ebenfalls möglich. Dabei wird der Kohlenhydratbaustein aus Beispiel I zuerst mit einem seitenkettengeschützten Lysin-Derivat verknüpft:

### 8a) N^{α}-[O-(3-O-Methyl-β-L-fucopyranosyl)-4-hydroxyphenylamino-thiocarbonyl]-N^{ε}-[fluorenyl-9-methoxycarbonyl]L-lysin

Eine Lösung von 6,8 g (25,3 mmol) p-Aminophenyl-3-O-methyl-β-L-fucopyranosid (Beispiel I) in 600 ml Dioxan/Wasser 1:1 wird unter Rühren mit 2,72 ml Thiophosgen (1,4 Äq.) versetzt. Nach 10 min. versetzt man mit 26 ml Ethyldiisopropylamin, rührt weitere 5 min bei RT und engt anschließend im Vakuum auf ein Volumen von 150 ml ein. Man setzt 800 ml Dichlormethan zu und trennt die Phasen. Die organische Phase wird zweimal mit Wasser gewaschen, über Natriumsulfat getrocknet und eingeengt. Der Rückstand wird mit 200 ml Methyl-tert.butylether und 200 ml Petrolether verrührt und abgesaugt. Man erhält 7,26 g (92 %) des Isothiocyanats.

2,92 g (9,4 mmol) des erhaltenen Isothiocyanats werden in 200 ml Dioxan/Wasser 1:1 gelöst und mit 3,11 g (0,9 Äq) von N^{ε}-[Fluorenyl-9-methoxycarbonyl]-lysin sowie 3,2 ml Ethyl-diisopropylamin versetzt. Man rührt für 16 h bei Raumtemperatur, engt dann im Vakuum ein und nimmt den Rückstand mit Wasser auf Durch Einstellen des pH-Werts auf 2 mit 1 N HCl wird das Produkt ausgefällt und nach 30 min abgesaugt. Der Rückstand wird in Dichlormethan suspendiert und das Lösungsmittel zweimal abgezogen, Nach mehrmaligem Waschen mit Ether und Petrolether erhalt man 5,3 g (92 %) Zielproduktes [DC: Acetonitril/Wasser/Eisessig 5:1:0,2 R_{f}= 0,69].

### 8b) 20(S)-20-O-{N^{α}-[O-(3-O-Methyl-β-L-fucopyranosyl)-4-hydroxy-phenylamino-thiocarbonyl]-N^{ε}-[fluorenyl-9-methoxycarbonyl]-L-lysyl-L-valyl}-camptothecin

1,2 g (1,76 mmol) N^{α}-[O-(3-O-Methyl-β-L-fucopyranosyl)-4-hydroxy-phenylaminothiocarbonyl]-N^{ε}-[fluorenyl-9-methoxycarbonyl]-L-lysin (Beispiel 8a) und 675 mg (1,2 mmol) der Verbindung III.b)L werden in 50 ml Dimethylformamid gelöst, die Mischung auf 0°C abgekühlt und anschließend mit 346 mg (1,8 mmol) N-Ethyl-N'-(dimethylaminopropyl)-carbodiimid Hydrochlorid versetzt. Unter Rühren läßt man die Temperatur über Nacht auf RT ansteigen. Das Lösungsmittel wird i. vac. abgedampft und der Rückstand mit Wasser verrührt. Man reinigt durch Flash-Chromatographie an Kieselgel, wobei man mit Acetonitrol als Laufmittel startet und später auf Acetonitril/Wasser 50:1 übergeht. Nach Einengen der entsprechenden Fraktionen erhält man 1,06 g (76 %) des Zielprodukts [DC: Acetonitril/Wasser 20:1 R_{f}= 0,34].

Die Deblockierung und die anschließende Überführung in das Hydrochlorid erfolgt in Analogie zu Beispiel 1.

### Beispiel 9:

### 20(S)-20-O-{N^{α}-[O-(3-O-Methyl-β-L-fucopyranosyl)-4-hydroxy-phenylaminothiocarbonyl]-L-histidyl-L-valyl}-camptothecin, Hydrochlorid,:

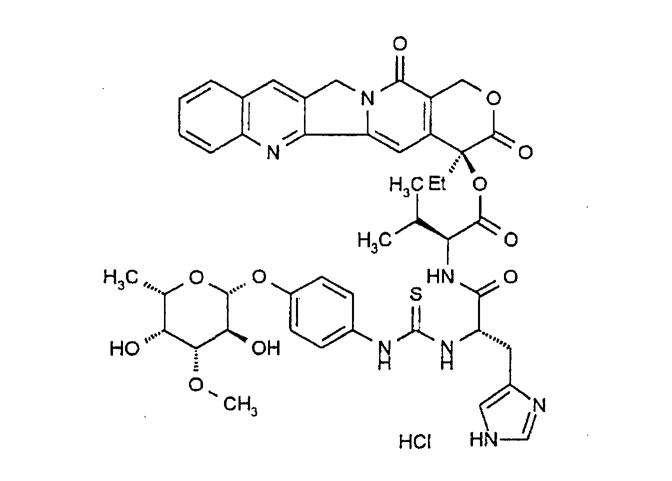

Die Verbindung wird ausgehend von dem unpolaren Diastereomer von 20-O-Valyl-Camptothecin, Trifluoracetat (III.b) L in Analogie zu Beispiel 2 hergestellt.
Das Diastereomerenverhältnis kann durch analytische HPLC ermittelt werden. Gegebenenfalls durch Kristallisation aus Methanol kann eine weitere Aufreinigung des unpolaren Diastereomers mit L-Konfiguration des Valins erreicht werden (>20:1)
HPLC-Bedingungen:
Trennsäule: Macherey & Nagel 250 x 4 mm Nucleosil 100-7 C 18 AB
Laufmittel A: H₂O + 0,1M KH₂PO₄
Laufmittel B: Acetonitril / Wasser 4:1 + 0,02M KH₂PO₄
Fluß: 1 ml/min
Inj. -Volumen: 15 µl

Im ¹H-NMR-Spektrum zeigt das reine Diastereomer nur einen Signalsatz z.B. für die beiden Singuletts der aromatischen H-Atome im Camptothecin-B-Ring bzw. D-Ring.
Diastereomer mit L-Valin:

| | |
|---|---|
| 400-MHz-¹H-NMR (CD₂Cl₂/CD₃OD 1:1): δ | s C-H (B-Ring) 8,58 ppm (überlagert von einem CH arom. von Histidin) |
| | s C-H (D-Ring) 7,35 ppm |
| [DC: Acetonitril/Wasser/Eisessig 5:1:0,2 Rf = 0,36] [FAB-MS: m/z = 896 (M + H⁺)]. | |

### Beispiel 10:

### 20(S)-20-O-{N^{α}-[O-(3-O-Methyl-β-L-fucopyranosyl)-4-hydroxy-phenylaminothiocarbonyl]-L-arginyl-L-leucyl}-camptothecin;

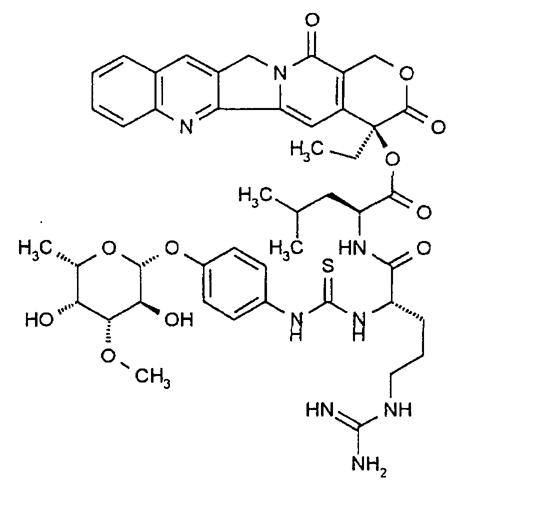

Das Produkt wird in Analogie zu Beispiel 5 hergestellt. Dabei wird auf der Stufe des 20-O-Leucyl-Camptothecins II.b das unpolare Diastereomer II.b) L eingesetzt.
[DC: Acetonitril/Wasser/Eisessig 5:1:0,2 R_{f}= 0,4] [FAB-MS: m/z = 929 = M+H⁺]. Mit den unter Beispiel 8 und 9 angegebenen HPLC-Bedingungen kann man die Diastereomeren aus Beispiel 5 und 10 unterscheiden.

## Patentansprüche

1. Camptothecin-Glycokonjugate der Formel (I) 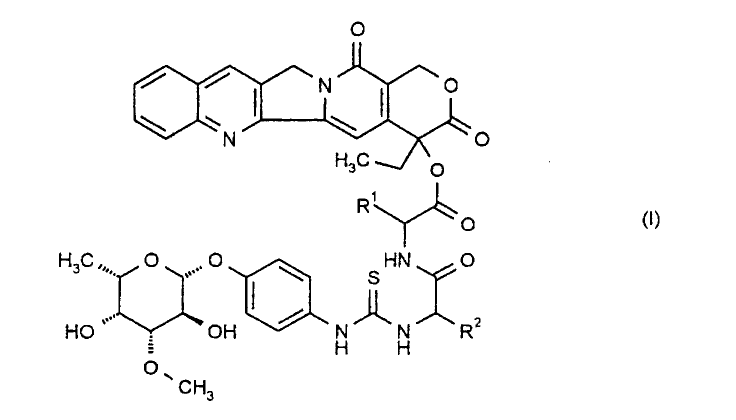 worin
R¹ für eine sterisch anspuchsvolle unpolare Seitenkette einer Aminosäure steht und
R² für eine basische Seitenkette einer Aminosäure steht
sowie deren Salze, Stereoisomere und Stereoisomerengemische.

2. Verbindungen der Formel (I) gemäß Anspruch 1, wobei
R¹ für einen verzweigten Alkylrest mit bis zu 4 Kohlenstoffatomen steht und
R² für einen Rest der Formel -(CH₂)ₙ-R³ steht, wobei
R³ -NH₂, 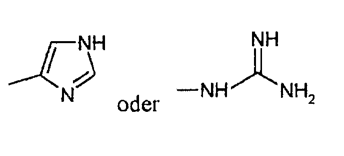 bedeutet und
n für eine Zahl 1 bis 4 steht,
und deren Salze, Stereoisomeren und Stereoisomerengemische.

3. Verbindungen der Formel (I) gemäß Anspruch 1, wobei
R¹ für einen verzweigten Alkylrest der Formeln 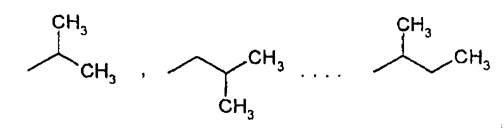 steht und
R² für einen Rest der Formeln -(CH₂)₂-NH₂, -(CH₂)₃-NH₂, -(CH₂)₄-NH₂, 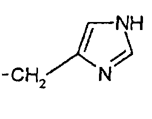 oder 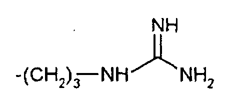 steht,
und deren Salze, Stereoisomeren und Stereoisomerengemisch.

4. Verfahren zur Herstellung der Glycokonjugate der Formel (I) 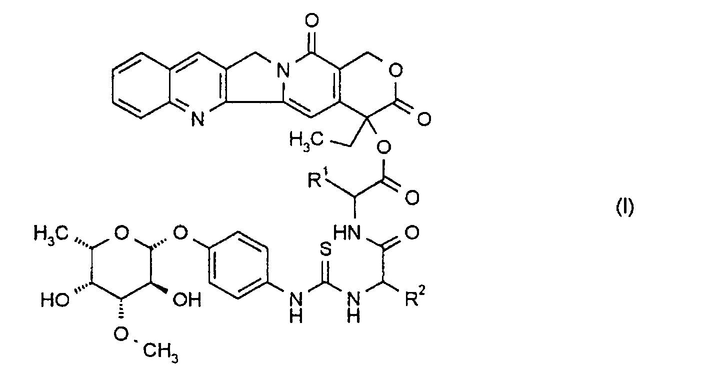 worin
R¹ für eine sterisch anspruchsvolle unpolare Seitenkette einer Aminosäure steht und
R² für eine basische Seitenkette einer Aminosäure steht,
oder von deren Salzen, **dadurch gekennzeichnet, daß** man das Isothiocyanat der Formel (II) 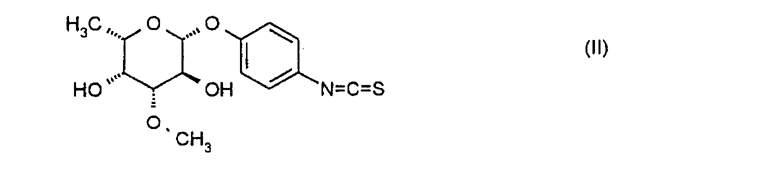 mit dem gegebenenfalls in der Seitenkette eine Schutzgruppe tragenden Peptidyl-Camptothecin der Formel (III) 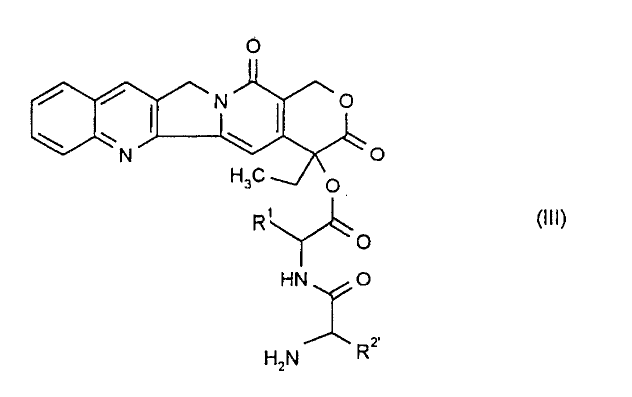 worin
R¹ die obengenannte Bedeutung hat und
R^{2'} die Bedeutung des obengenannten basischen Rests R² hat, der darüberhinaus an der basischen Gruppe eine in der Peptidchemie übliche Schutzgruppe tragen kann
zu dem Glycokonjugat der Formel (IV) 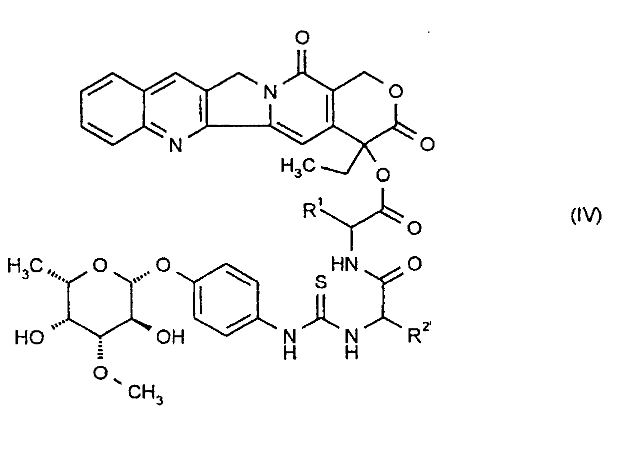 worin
R¹ und R^{2'} die oben angegebenen Bedeutungen haben,
umsetzt,
die gegebenenfalls vorhandene Seitenketten-Aminoschutzgruppe nach üblichen Methoden abspaltet und
die erhaltene Verbindung gegebenenfalls in das gewünschte Salz überführt.

5. Verfahren zur Herstellung von Verbindungen der allgemeinen Formel (I) oder von deren Salzen, **dadurch gekennzeichnet, daß** man das Isothiocyanat der Formel (II) 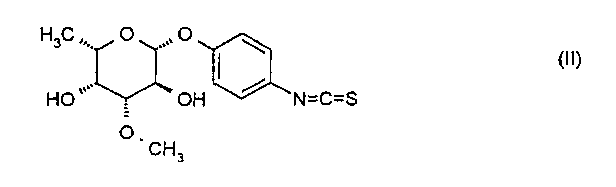 mit einer gegebenenfalls geeignet geschützten terminalen basischen Aminosäure der Formel (V) 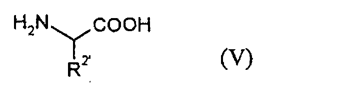 worin R^{2'} für eine basische Seitenkette einer Aminosäure steht, deren basische Gruppe geschützt sein kann,
zu einem Aminosäurekonjugat der Formel (VI) 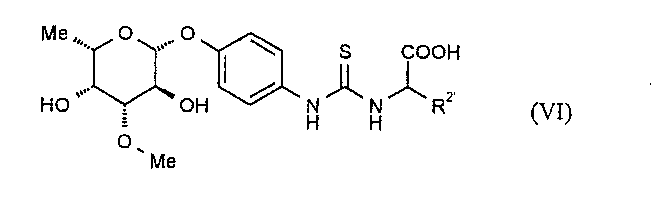 worin R^{2'} die vorstehend angegebene Bedeutung hat, umsetzt,
diese dann mit Aminosäurekonjugaten der Formel (VII) 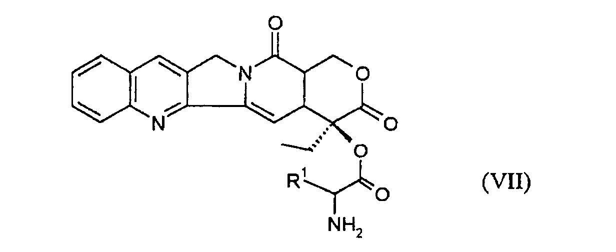 worin R¹ die in Anspruch 1 angegebene Bedeutung hat,
umsetzt,
die Seitenkettenschutzgruppe gegebenenfalls abspaltet und die Verbindungen gegebenenfalls in ein geeignetes Salz überführt.

6. Verbindung gemäß Anspruch 1, **dadurch gekennzeichnet, daß** es sich um 20(S)-20-O-{N^{α}-[O-(3-O-Methyl-β-L-fucopyranosyl)-4-hydroxy-phenylamino-thiocarbonyl]-L-lysyl-L-valyl}-camptothecin der Formel 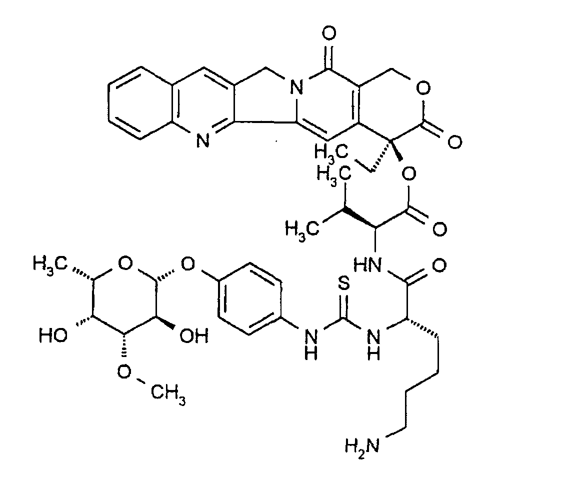 und dessen Salze handelt.

7. Verbindung gemäß Anspruch 1, **dadurch gekennzeichnet, daß** es sich um 20(S)-20-O-{N^{α}-[O-(3-O-Methyl-β-L-fucopyranosyl)-4-hydroxy-phenylamino-thiocarbonyl]-L-histidyl-L-valyl}-camptothecin der Formel 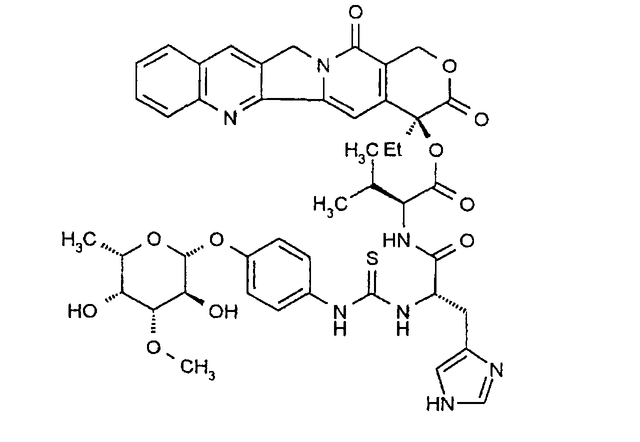 und dessen Salze handelt.

8. Verwendung von Verbindungen der Formel (I) gemäß Anspruch 1 zur Herstellung von Arzneimitteln.

9. Arzneimittel enthaltend Verbindungen der Formel (I) gemäß Anspruch 1.

## Claims

1. Camptothecin glycoconjugates of the formula (I) 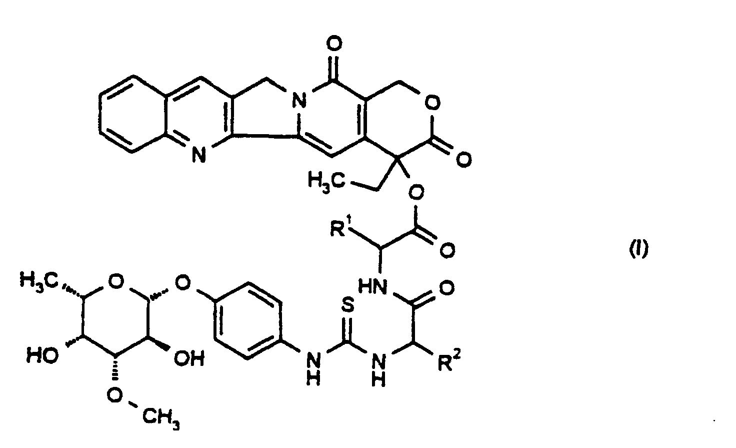 in which
R¹ represents a sterically demanding non-polar side chain of an amino acid and
R² represents a basic side chain of an amino acid
and their salts, stereoisomers and stereoisomer mixtures.

2. Compounds of the formula (I) according to Claim 1, where
R¹ is a branched alkyl radical having up to 4 carbon atoms and
R² is a radical of the formula -(CH₂)ₙ-R³, where
R³ is 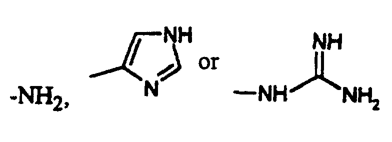 and
n is a number 1 to 4,
and their salts, stereoisomers and stereoisomer mixtures.

3. Compounds of the formula (I) according to Claim 1, where
R¹ is a branched alkyl radical of the formula 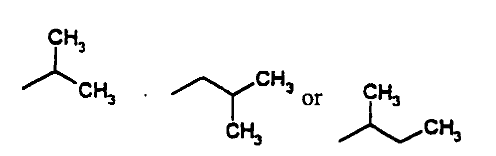 and
R² is a radical of the formula 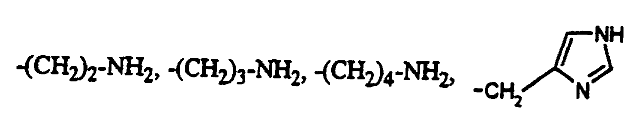 or 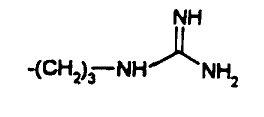
and their salts, stereoisomers and stereoisomer mixtures.

4. Process for the preparation of the glycoconjugates of the formula (I) 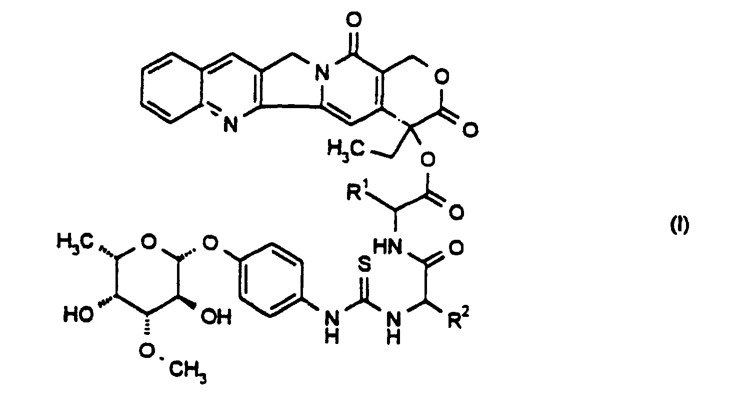 in which
R¹ represents a sterically demanding non-polar side chain of an amino acid and
R² represents a basic side chain of an amino acid,
or of their salts, **characterized in that** the isothiocyanate of the formula (II) 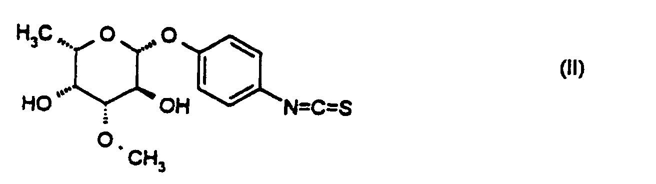 is reacted with the peptidyl-camptothecin, optionally bearing a protective group in the side chain, of the formula (III) 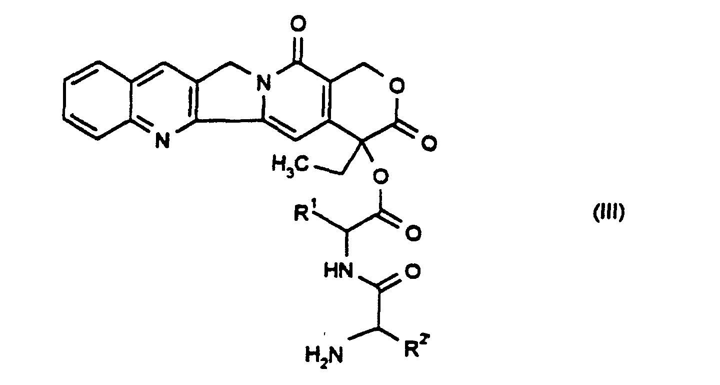 in which
R¹ has the abovementioned meaning and
R^{2'} has the meaning of the abovementioned basic radical R², which moreover can carry a protective group customary in peptide chemistry on the basic group
to give the glycoconjugate of the formula (IV) 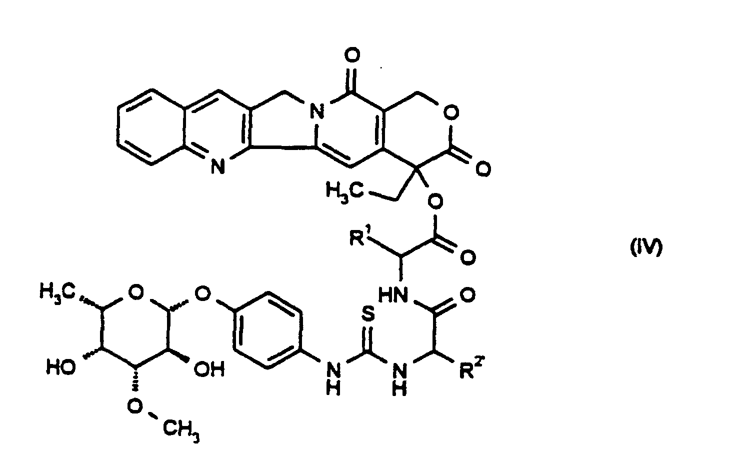
in which
R¹ and R^{2'} have the meanings indicated above,
the side chain amino protective group which may be present is removed according to customary methods and
the compound obtained is optionally converted into the desired salt.

5. Process for the preparation of compounds of the general formula (I) or of their salts, **characterized in that** the isothiocyanate of the formula (II) 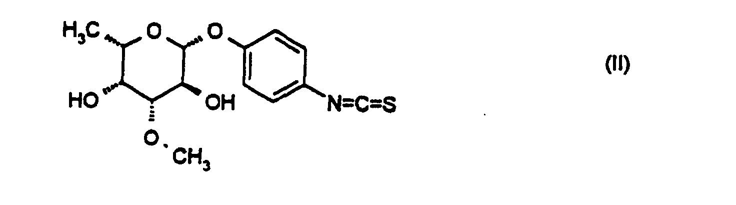 is reacted with an optionally suitably protected terminal basic amino acid of the formula (V) 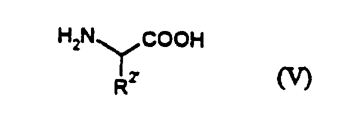 in which R^{2'} represents a basic side chain of an amino acid whose basic group can be protected,
to give an amino acid conjugate of the formula (VI) 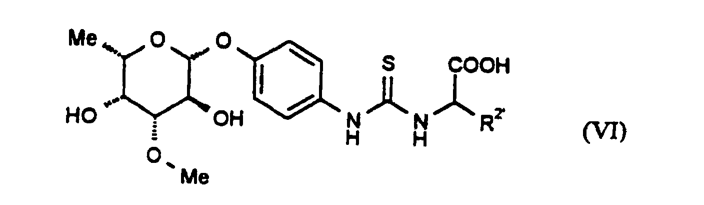 in which R^{2'} has the meaning indicated above,
this is then reacted with amino acid conjugates of the formula (VII) 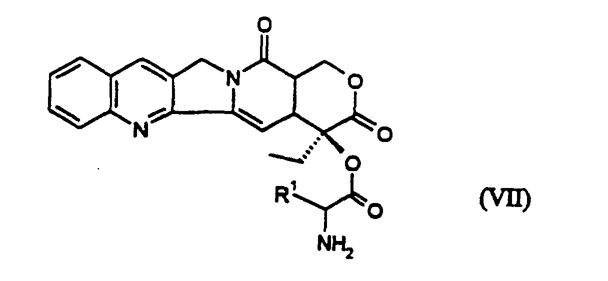 in which R¹ has the meaning indicated in Claim 1,
the side chain protective group is optionally removed and the compounds are optionally converted into a suitable salt.

6. Compound according to Claim 1, **characterized in that** it is 20(S)-20-O-{N^{α}-[O-(3-O-methyl-β-L-fucopyranosyl)-4-hydroxy-phenylamino-thiocarbonyl]-L-lysyl-L-valyl}-camptothecin of the formula 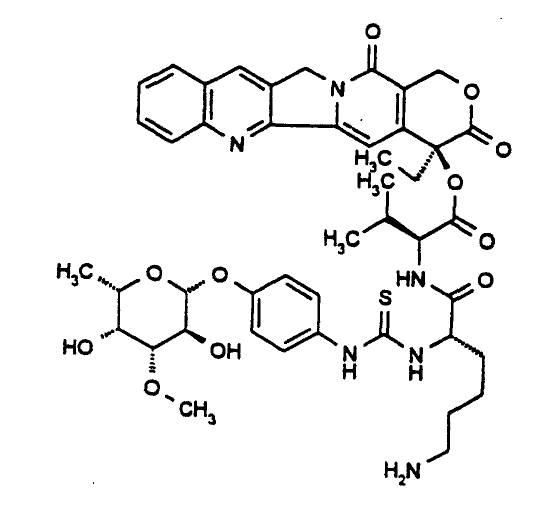 and its salts.

7. Compound according to Claim 1, **characterized in that** it is 20(S)-20-O-{N^{α}-[O-(3-O-methyl-β-L-fucopyranosyl)-4-hydroxy-phenylamino-thiocarbonyl]-L-histidyl-L-valyl}-camptothecin of the formula 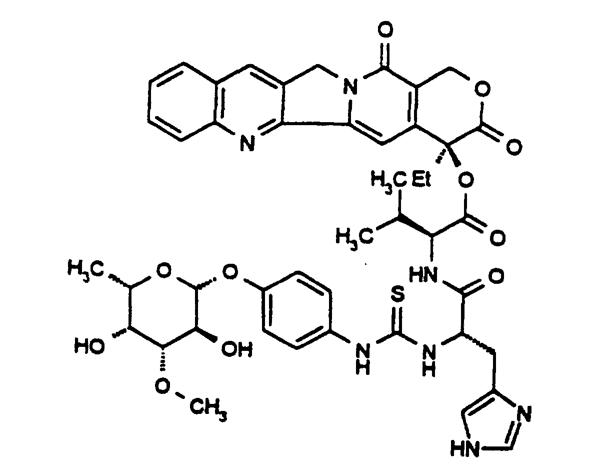 and its salts.

8. Use of compounds of the formula (I) according to Claim 1 for the production of medicaments.

9. Medicaments comprising compounds of the formula (I) according to Claim 1.

## Revendications

1. Glycoconjugués de camptcthécine de formule (I) 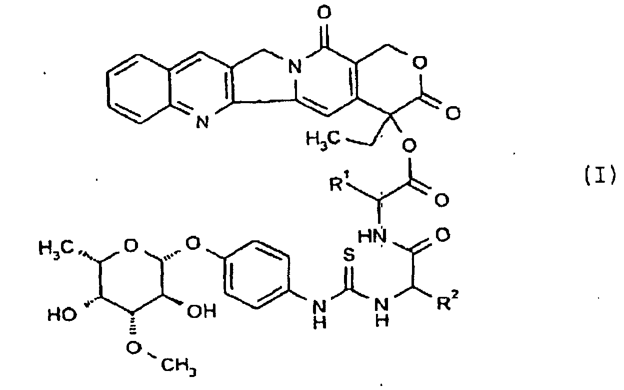 dans laquelle
R¹ est une chaîne latérale non polaire stériquement exigeante d'un amino-acide et
R² est une chaîne latérale basique d'un amino-acide, ainsi que leurs sels, leurs stéréo-isomères et leurs mélanges de stéréo-isomères.

2. Composés de formule (I) suivant la revendication 1, dans lesquels
R¹ désigne un reste alkyle ramifié ayant jusqu'à 4 atomes de carbone et
R² désigne un reste de formule -(CH₂)ₙ-R³, dans laquelle -NH₂, 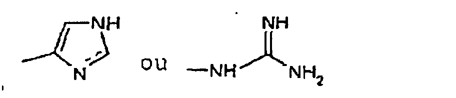
R³ est un groupe et
n est un nombre de 1 à 4,
et leurs sels, leurs stéréo-isomères et leurs mélanges de stéréo-isomères.

3. Composés de formule (I) suivant la revendication 1, dans lesquels
R¹ est un reste alkyle ramifié de formules 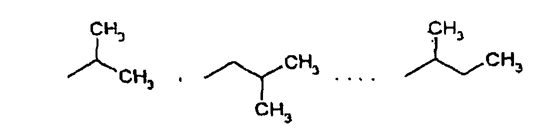 et
R² est un reste de formules 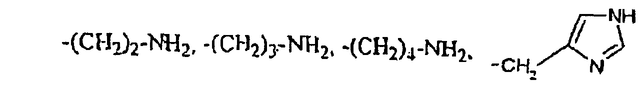 ou 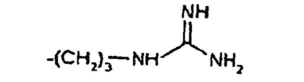
et leurs sels, leurs stéréo-isomères et leurs mélanges de stéréo-isomères.

4. Procédé de production des glycoconjugués de formule (I) 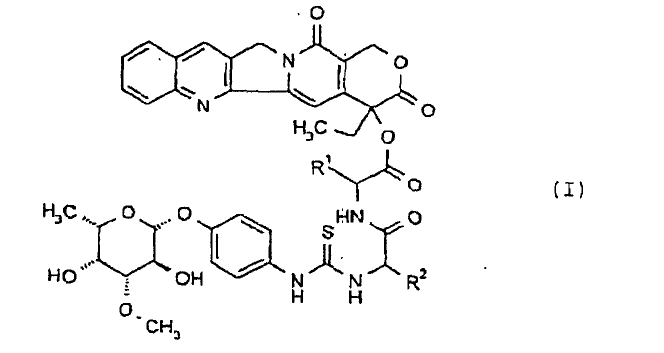 dans laquelle
R¹ désigne une chaîne latérale non polaire stériquement exigeante d'un amino-acide et
R² désigne une chaîne latérale basique d'un amino-acide,
ou de leurs sels, **caractérisé en ce qu'**on fait réagir l'isothiocyanate de formule (II) 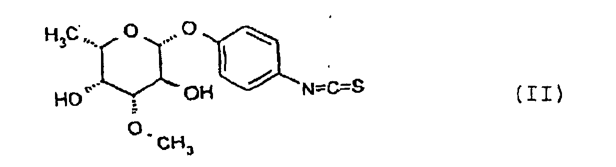 avec la peptidylcamptothécine de formule (III) portant éventuellement un groupe protecteur en chaîne latérale, 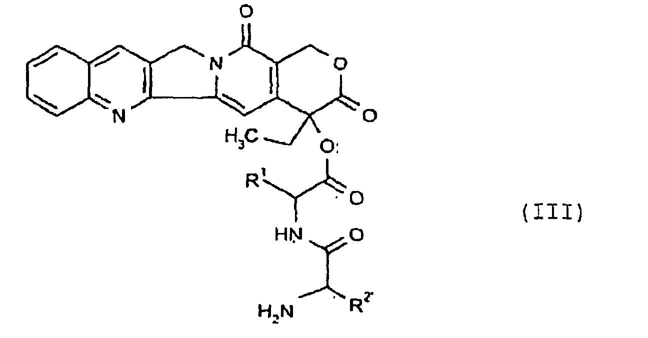 formule dans laquelle
R¹ a la définition indiquée ci-dessus et
R² a la définition du reste basique R² mentionné ci-dessus, qui peut en outre porter sur le groupe basique un groupe protecteur classique dans la chimie des peptides
pour former le glycoconjugué de formule (IV) 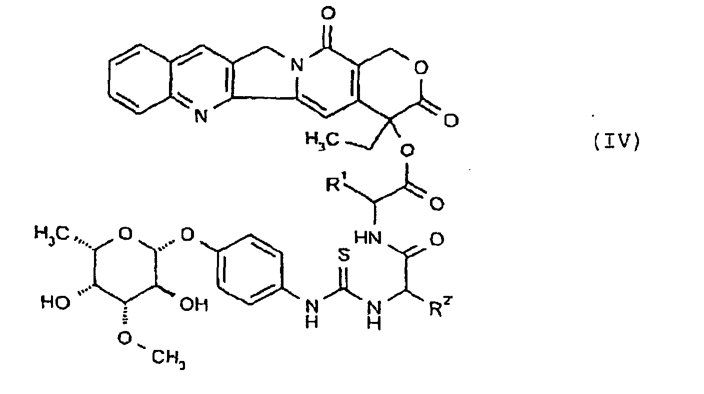
dans laquelle
R¹ et R² ont les définitions indiquées ci-dessus,
on élimine par des modes opératoires classiques le groupe éventuellement présent protégeant le groupe amino en chaîne latérale et
on transforme éventuellement le composé obtenu en le sel souhaité.

5. Procécé de production de composés de formule générale (I) ou de leurs sels, **caractérisé en ce qu'**on fait réagir l'isothiocyanate de formule (II) 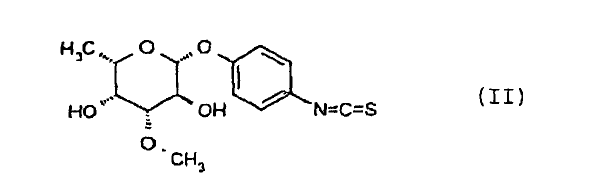 avec un amino-acide basique terminal le cas échéant convenablement protégé, de formule (V) 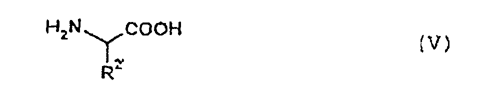 dans laquelle R^{2'} désigne une chaîne latérale basique d'un amino-acide dont le groupe basique peut être protégé,
pour former un conjugué d'amino-acide de formule (VI) 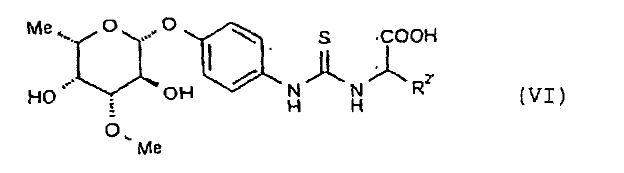 où R^{2'} a la définition indiquée ci-dessus,
on fait ensuite réagir ce conjugué avec des conjugués d'amino-acide de formule (VII) 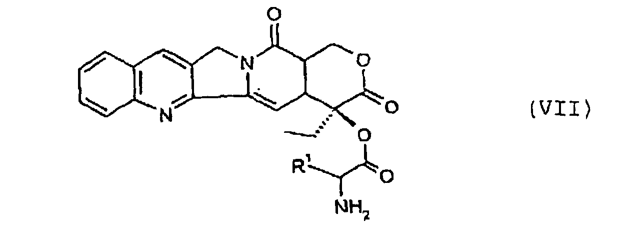 dans laquelle R¹ a la définition indiquée dans la revendication 1,
on élimine éventuellement le groupe protecteur en chaîne latérale et on transforme éventuellement les composés en un sel convenable.

6. Composé suivant la revendication 1, **caractérisé en ce qu'**il s'agit de la 20(S)-20-O-{N^{α}-[O-(3-O-méthyl-β-L-fucopyrannosyl)-4-hydroxy-phényl-amino-thiocarbonyl]-L-lysyl-L-valyl}-camptothécine de formule 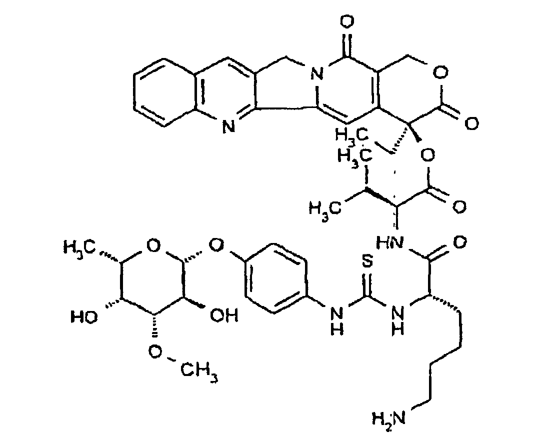 et de ses sels.

7. Composé suivant la revendication 1, **caractérisé en ce qu'**il s'agit de la 20(S)-20-O-{N^{α}-[O-(3-O-méthyl-β-L-fucopyrannosyl)-4-hydroxy-phényl-amino-thiocarbonyl]-L-histidyl-L-valyl}-camptothécine de formule 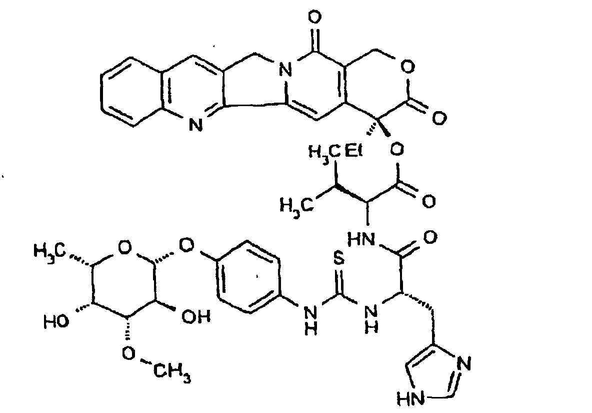 et de ses sels.

8. Utilisation de composés de formule (I) suivant la revendication 1 pour la préparation de médicaments.

9. Médicaments ccntenant des composés de formule (I) suivant la revendication 1.
